(19) **Europäisches Patentamt**
**European Patent Office**
**Office européen des brevets**

(11) **EP 1 581 246 B1**

(12) **EUROPEAN PATENT SPECIFICATION**

(45) Date of publication and mention
of the grant of the patent:
**16.01.2013 Bulletin 2013/03**

(51) Int Cl.:
*A61K 38/22* [(2006.01)]          *A61K 38/26* [(2006.01)]

(21) Application number: **03813809.5**

(86) International application number:
**PCT/US2003/040713**

(22) Date of filing: **19.12.2003**

(87) International publication number:
**WO 2004/056317 (08.07.2004 Gazette 2004/28)**

(54) **COMPOSITIONS FOR THE TREATMENT AND PREVENTION OF NEPHROPATHY**

ZUSAMMENSETZUNGEN ZUR BEHANDLUNG UND PREVENTION VON NEPHROPATHIE

COMPOSITIONS DESTINÉES AU TRAITEMENT ET A LA PRÉVENTION DE LA NEPHROPATHIE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **19.12.2002 US 434888 P**
**17.12.2003 US 740146**

(43) Date of publication of application:
**05.10.2005 Bulletin 2005/40**

(73) Proprietors:
• **Amylin Pharmaceuticals, LLC**
**San Diego, CA 92121 (US)**
• **AstraZeneca Pharmaceuticals LP**
**Wilmington, DE (US)**

(72) Inventors:
• **BARON, Alain, D.**
**San Diego, CA 92130 (US)**

• **HATHAWAY, David, R.**
**Lincoln, NE 68516 (US)**
• **MISTRY, Mahesh**
**Pepperell, MA 01463 (US)**
• **ROMAN, Richard, J.**
**Brookfield, WI 53005 (US)**

(74) Representative: **Raynor, Stuart Andrew et al**
**J A Kemp**
**14 South Square**
**Gray's Inn**
**London WC1R 5JJ (GB)**

(56) References cited:
**WO-A-01/27128          WO-A-02/40448**
**WO-A-99/40788          WO-A-02/083066**
**US-A1- 2001 016 586     US-A1- 2002 107 206**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 1 581 246 B1

**Description**

## FIELD OF THE INVENTION

This invention relates to a composition and it use in a method of treating nephropathy, and especially hypertensive and diabetic nephropathy, using GLP-1 and related compounds.

## BACKGROUND OF THE INVENTION.

End stage renal disease (ESRD) is a major health problem in the United States. The incidence rate has steadily increased over the past decade, from 155 per million population in 1988 to 296 in 1997. The disease is especially prevalent in racial and ethnic minorities; specifically African Americans, American Indians, Alaskan natives, Native Hawaiians and other Pacific Islanders, and Hispanic Americans.

The four major causes of ESRD include diabetes mellitus (primarily type-2), hypertension, glomerulonephritis, and cystic renal disease. There is significant variability in the cause of ESRD among the various ethnic and racial groups. For instance, whereas diabetic nephropathy is the predominant cause of ESRD in American Indians/Alaskan Natives, Asian Americans, Native Hawaiians and other Pacific Islanders, Hispanic Americans, and Caucasians, hypertensive nephropathy is the most frequently reported cause of ESRD in African Americans.

Currently, patients with ESRD must either go on dialysis or receive a new kidney through transplant. Every year, high blood pressure causes more than 15,000 new cases of ESRD in the United States.

Diabetic neuropathy is kidney disease that develops as a result of diabetes mellitus. Diabetes affects approximately 5% of the U.S. population. Approximately 25-40% of patients with diabetes ultimately develop diabetic nephropathy, which progresses through five predictable stages, the final stage of which is ESRD, whereby renal replacement therapy (i.e., hemodialysis, peritoneal dialysis, kidney transplantation) is necessary.

Hypertension and diabetes often coexist in the same patient acting synergistically. The underlying mechanism for nephropathy is not fully understood, but has been postulated to involve a period of glomerular hyperemia followed by a reactive vasoconstriction, leading to glomerular hypertension and subsequent injury. An early manifestation of nephropathy is protein in the urine (e.g., proteinuria), the concentration of which may relate to the degree of kidney damage. Eventually, glomerulosclerosis occurs, leading to a progressive loss of functioning nephrons. The capacity of the kidneys to filter/secrete waste products and maintain electrolyte and warer balance is lost, with a a rise in the serum creatinine and Blood Urea Nitrogen (BUN) as well as accumulation of excess fluid. At this stage, patients are generally diagnosed with end state renal disease (ESRD).

Individuals with insulin resistance are also at risk, whether or not they have co-existing hypertension, as are patients having so-called "metabolic syndrome.

The rate of progression of nephropathy can be forestalled by treatment with angiotensin-converting enzyme inhibitors or with the calcium channel blocking drug, verapamil. However, ESRD is inevitable. ESRD, progressing to renal failure, can be treated by dialysis or kidney transplantation. These are expensive therapies that are currently reimbursed by Medicare (irrespective of patient age) with an annual cost of $10 billion. The prevalence of ESRD is increasing.

Accordingly, it can be seen that there is a real and continuing need for an effective treatment for renal damage and nephropathy, including that occurring in conjunction with hypertension, insulin resistance, and/or diabetes. This invention has as its primary object the fulfillment of this need.

The present invention provides the use of a GLP-1, an exendin, or an agonist analog having at least 70% amino acid sequence identity with GLP-1 (7-36) or exendin-4, in the manufacture of a medicament for the prevention or treatment of nephropathy.

The present invention also provides the use of a GLP-1, an exendin, or an agonist analog having at least 70% amino acid sequence identity with GLP-1 (7-36) or exendin-4, in the manufacture of a medicament for preventing progression of nephropathy to ESRD in a subject having nephropathy.

The present invention further provides the use of a GLP-1, an exendin, or an agonist analog having at least 70% amino acid sequence identity with GLP-1 (7-36) or exendin-4, in the manufacture of a medicament for reducing proteinuria in a subject in need thereof.

The present invention additionally provides the use of a GLP-1, an exendin, or an agonist analog having at least 70% amino acid sequence identity with GLP-1 (7-36) or exendin-4, in the manufacture of a medicament for preventing or slowing progression of glomerulosclerosis in a subject in need thereof.

In a preferred embodiment, the subject also suffers from insulin resistance, diabetes, or hypertension.

In one embodiment, the the GLP-1 is GLP-1 (7-36) NH$_2$.

In another embodiment, the exendin is exendin-3 or exendin-4.

Preferably, the composition is administered in a dose of from about 0.001 pmol/kg to 20 nmol/kg.

2

The invention also provides a GLP-1, an exendin, or an agonist analog having at least 70% amino acid sequence identity with GLP-1 (7-36) or exendin 4, for use in:

a) the prevention or treatment of nephropathy;
b) preventing progression of nephropathy to ESRD in a subject having nephropathy;
c) reducing proteinuria in a subject in need thereof; or
d) preventing or slowing progression of glomerulcsclerosis in a subject in need thereof.

The invention describes compositions and methods for use in the prevention and treatment of nephropathy, including hypertensive and diabetic nephropathy, and nephropathy associated with insulin resistance and metabolic syndrome. The invention achieves these ends by improving or preventing worsening of hypertension, endothelial function, renal function, and glomerulosclerosis, among other things.

In one embodiment, the invention provides the use of the claim compounds in a method for preventing or treating nephropathy, including hypertensive and diabetic nephropathy, or that related to insulin resistance, comprising administering a compound of the invention.

The invention further provides the use of the claim compounds in methods for improving endothelial function in a patient having reduced vasodilatory capacity; or having glomerulosclerosis or any other reduction in glomerular flow. Such improvement in endothelial function serves both to reduce hypertension and to improve the function of the capillaries of the glomeruli. In additional embodiments, the molecules of the invention are useful to prevent progression of nephropathy to ESRD, to prevent, slow the progression of, treat or ameliorate proteinuria and/or glomerulosclerosis.

In preferred embodiments of the invention, the compound is a GLP-1 or exendin-3 or exendi-4, or a biologically active analog, derivative, variant, or fragment of them. Preferred dosages are from about 0.001 $\mu$g/kg/dose to about 1.0 $\mu$g/kg/dose, or at a dose sufficient to achieve a therapeutic plasma level of at least 40 pg/mL

The means and manner of accomplishing each of the above objectives will become apparent from the detailed description of the invention which follows hereinafter.

## BRIEF DESCRIPTION OF THE DRAWINGS

Figure 1 presents the effect of rGLP-1 on mean arterial pressure (MAP) in Dahl S rats.
Figure 2A presents the effect of rGLP-1 on proteinuria concentration in Dahl S rats.
Figure 2B presents the effect of rGLP-1 on microalbumimuria concentration in Dahl S rats.
Figure 2C presents the effect of rGLP-1 on plasma creatinine concentration in Dahl S rats.
Figure 3A presents the effect of rGLP-1 on kidney weight in Dahl S rats.
Figure 3B presents the effect of rGLP-1 on glomerular injury in Dahl S rats.
Figure 3C presents the effect of rGLP-1 on the formation of protein casts in outer medulla in Dahl S rats.
Figure 4 shows the effect of GLP-1 on endothelial function in aortic rings.

## DETAILED DESCRIPTION OF THE INVENTION

The present invention relates to compositions and their uses for the treatment of hypertensive, diabetic, and other types of nephropathy, such as analgesic nephropathy, IgA-nephropathy, ischemic nephropathy, HIV-associated nephropathy, membranous nephropathy, glomerulosclerosis, etc. The invention is especially effective for use in preventing or treating hypertensive and/or diabetic nephropathies, and those occurring or likely to occur in insulin resistant patients with or without co-existing hypertension.

Without wishing to be bound by theory, it is thought that the molecules of the invention act in part by improving insulin resistance, cation balance, hypertension, and/or by facilitating glucose oxidation by cells (including endothelial cells) in the kidney (and elsewhere) rather than oxidation of free fatty acids, leading to an enhanced production of ATP for use by the cell, and reduced oxidative stress on the affected tissue.

Molecules of the invention include compounds that binds to a receptor for the glucagon like peptide-1, glucagon-like peptide-1s (GLP-1), exendins, or agonist analogs.

As used herein, an "analog" includes any peptide whose sequence was derived from that of the base molecule (e.g., receptor-binding compound, incretin, GLP-1, or exendin), whether or not including insertions, substitutions, extensions, or deletions, preferably having at least 70%. amino acid sequence identity with the base molecule, more preferably having at least 80%, 90%, or 95% amino acid sequence identity with the base molecule. Such analogs may comprise conservative or non-conservative amino acid substitutions (including non-natural amino acids and L and D forms). An "agonist analog," is an analog that exhibits at least one characteristic or action of the base molecule, preferably having a potency better than the base molecule, or within five orders of magnitude (plus or minus) of potency compared to the base molecule, more preferably 4, 3, 2, or 1 order of magnitude, when evaluated by art-known measures such as receptor binding/competition studies.

An example of a base molecule of the invention, as the term is used above, is GLP-1, also known as glucagon-like peptide-1 [7-36], whether or not amided (often GLP-1 [7-36]NH$_2$), a product of the proglucagon gene having the amino acid sequence His Ala Glu Gly Thr Phe Thr Ser Asp Val Ser Ser Tyr Leu Glu Gly Gln Ala Ala Lys Glu Phe Ile Ala Trp Leu Val Lys Gly. Arg-[optional NH$_2$] (SEQ ID NO: 1). GLP-1 is a hormone produced by L-type cells in the intestine, and is released following ingestion of a meal. GLP-1 improves insulin resistance and glucose utilization in patients with type-2 diabetes by increasing the secretion of insulin and by inhibiting the secretion of glucagon. Receptors for GLP-1 are expressed in pancreatic islet cells, the gastrointestinal tract, and in the lung, heart, central nervous system and kidney. GLP-1 reportedly produces a variety of biological effects (e.g., Orskov, et al., Diabetes, 42:658-61, 1993; D'Alessio, et al., J. Clin. Invest., 97:133-38, 1996, Williams B, et al., J Clin Endocrinol Metab 81 (1): 327-32, 1996; Wettergren A, et al., Dig Dis Sci 38 (4): 665-73, 1993; Schjoldager BT, et al., Dig Dis Sci 34 (5): 703-8, 1989; O'Halloran DJ, et al., J Endocrinol 126 (1): 169-73, 1990; Wettergren A, et al., Dig Dis Sci 38 (4): 665-73, 1993). GLP-1[7-37], which has an additional glycine residue at its carboxy terminus, also stimulates insulin secretion in humans (Orskov, et al., Diabetes, 42:658-61, 1993).

Compositions of the invention include GLP-1 agonist analogs. By "agonist analog" is meant a compound that mimics at least one effect of GLP-1. This definition of agonist analog could include compounds that bind to a receptor or receptors where GLP-1 causes the particular effect. Certain GLP-1 analogs with agonist activity are described in Chen et al., U.S. Patent No. 5,512,549, issued April 30, 1996, entitled Glucagon-Like Insulinotropic Peptide Analogs, Compositions and Methods of Use. Other GLP-1 analogs with agonist activity are described in Johnson et al., U.S. Patent No. 5,574,008, issued November 12, 1996, entitled, Biologically Active Fragments of Glucagon-Like Insulinotropic Peptide. Still other GLP-1 analogs with agonist activity are described in Buckley et al., U.S. Patent No. 5,545,618, issued August 13, 1996, entitled GLP-1 Analogs Useful for Diabetes Treatment. The present invention includes the use of recombinant human GLP-1 analogs and GLP-1 analogs derived from other species, whether recombinant or otherwise synthetic.

In certain aspects, the GLP-1 agonist analogs used in the present invention can be GLP-1(7-34) and GLP-1 (7-35), as disclosed in U.S. Pat. No. 5,118,666, as well as GLP-1(7-37) as disclosed in U.S. Pat. No: 5,120,712. Also included are GLP-1 analogs having a reduced tendency to aggregate such as those described in WO 01/98331; GLP-1 analogs that have N-terminal truncation, US Patent No. 5,574,008; GLP-1 analogs with attached acyl groups, US Patent No. 5,512,549; and GLP-1 analogs that are amidated, WO 02/48192; and GLP-1 analogs of U.S. Patent Application Serial No. 10/276772.

Additional exemplary analogs include GLP-1 analogs modified at position 8, e.g., US Patent No. 5,981,488, incorporated by reference. In brief, analogs include those of formula (XI), R$_1$ X-Glu-Gly-Thr-Phe-Thr-Ser-Asp-Val-Ser-Ser-Tyr-Leu-Y-Gly-Gln-Ala-Ala-Lys-Z -Phe-Ile-Ala-Trp-Leu-Val-Lys-Gly-Arg-R$_2$ (SEQ ID NO:33) or a pharmacuetically accetable salt thereof, wherein:

   R$_1$ is selected from the group consisting of His, D-histidine, desamino-histidine, 2-amino-histidine, beta.-hydroxy-histidine, homohistidine, alpha-fluoromethyl-histidine, and alpha-methyl-histidine;
   X is selected from the group consisting of Met, Asp, Lys, Thr, Leu, Asn, Gln, Phe, Val, and Tyr
   Y and Z are independently selected from the group consisting of Glu, Gln, Ala, Thr, Ser, and Gly, and;
   R$_2$ is selected from the group consisting of NH$_2$, and Gly-OH; provided that, if R$_1$ is His, X is Val, Y is Glu, and Z is Glu, then R$_2$ is NH$_2$.

V8-GLP-1 and other position 8 analogs can be found in US Patent No. 5,705,483. In brief, analogs include those of formula (XII), R$_1$ X-Glu-Gly-Thr-Phe-Thr-Ser-Asp-Val-Ser-Ser-Tyr-Leu-Y-Gly-Gln-Ala-Ala-Lys-Z -Phe-Ile-Ala-Trp-Leu-Val-Lys-Gly-Arg-R$_2$ (SEQ ID NO: 34) wherein:

   R$_1$ is selected from the group consisting of L-histidine, D-histidine, desamino-histidine, 2-amino-histidine, beta-hydroxy-histidine, homohistidine, alpha-fluoromethyl-histidine, and alpha-methyl-histidine;
   X is selected from the group consisting of Ala, Gly, Val, Thr, Ile, and alpha-methyl-Ala;
   Y is selected from the group consisting of Glu, Gln, Ala, Thr, Ser, and Gly;
   Z is selected from the group consisting of Glu, Gln, Ala, Thr, Ser, and Gly;
   R$_2$ is selected from the group consisting of NH$_2$, and Gly-OH; providing that the compound has an isoelectric point in the range from about 6.0 to about 9.0 and further providing that when R$_1$ is His, X is Ala, Y is Glu, and Z is Glu, R$_2$ must be NH$_2$.

In other aspects, the GLP-1 agonist analogs are variants or analogs of GLP-1 known in the art, such as, for

example, Gln[9] -GLP-1(7-37), D-Gln[9] -GLP-1(7-37), acetyl-Lys[9] -GLP-1(7-37), Thr[16] -Lys[18] -GLP-1(7-37), and Lys[18] -GLP-1(7-37). Derivatives of GLP-1 are also contemplated in the present invention and include, for example, acid addition salts, carboxylate salts, lower alkyl esters, and amides (see, e.g., WO91/11457). Generally, but not necessarily for use in this invention, the various forms of GLP-1 are known to stimulate insulin secretion (insulinotropic action) and cAMP formation (see, e.g., Mojsov, S., Int. J. Peptide Protein Research, 40:333-343 (1992)).

In still other aspects, the present invention contemplates GLP-1 agonists of the general formula (I):

R$_1$-Ala-Glu-Gly-Thr-Phe-Thr-Ser-Asp-Val-Ser-Ser-Tyr-Leu-Glu-Gly-Gln-Ala-Ala-Xaa$_{40}$-Glu-Phe-Ile-Ala-Trp-Leu-

|

Val-Lys-Gly-Arg-R$_3$ (SEQ ID NO:2)        R$_2$

wherein R$_1$ is selected from the group consisting of 4-imidazopropionyl (des-amino-histidyl), 4-imidazoacetyl, or 4-imidazo-alpha, alpha dimethyl-acetyl;
R$_2$ is selected from the group consisting of C$_6$ -C$_{10}$ unbranched acyl, or is absent;
R$_3$ is selected from the group consisting of Gly-OH or NH$_2$; and,
Xaa$_{40}$ is Lys or Arg.

In one embodiment, the GLP-1 agonists are naturally-occurring GLP-1(7-37) that arise from adding various R groups via a peptide bond to the amino terminus of the peptide portion of Formula I (SEQ ID NO:2). Optionally, further compounds of the invention are made by acylating the epsilon amino group of the Lys34 residue and by making limited amino acid substitutions at position 26 or by altering the carboxy terminus.
It should be noted that for the above formula, the nomenclature scheme used is that which has been developed around processed forms of GLP-1. In this scheme, the amino terminus of the known GLP-1(7-37) OH has been assigned number 7 and the carboxy terminus number 37. Therefore, the first Ala residue of Formula I corresponds to residue 8 of GLP-1 (7-37)OH. Likewise Xaa$_{40}$ in Formula I corresponds to residue 26 of GLP-1(7-37)OH, and so forth.
In still other aspects, the present invention provides biologically-active GLP-1 fragments of formula (II):

R$_4$-Ser-Tyr-Leu-Glu-Gly-Gln-Ala-Ala-Lys-Glu-Phe-Ile-Ala-Trp-Leu-Val-Xaa$_{41}$-Gly-Arg -R$_5$ (SEQ ID NO:3)
wherein R$_4$ is selected from the group consisting of:

a) H$_2$ N;
b) H$_2$ N-Ser;
c) H$_2$ N-Val-Ser;
d) H$_2$ N-Asp-Val-Ser;
e) H$_2$ N-Ser-Asp-Val-Ser (SEQ ID NO:4);
f) H$_2$ N-Thr-Ser-Asp-Val-Ser (SEQ ID NO:5);
g) H$_2$ N-Phe-Thr-Ser-Asp-Val-Ser (SEQ ID NO:6);
h) H$_2$ N-Thr-Phe-Thr-Ser-Asp-Val-Ser (SEQ ID NO:7);
i) H$_2$ N-Gly-Thr-Phe-Thr-Ser-Asp-Val-Ser (SEQ ID NO:8);
j) H$_2$ N-Glu-Gly-Thr-Phe-Thr-Ser-Asp-Val-Ser (SEQ ID NO:9); or
k) H$_2$ N-Ala-Glu-Gly-Thr-Phe-Thr-Ser-Asp-Val-Ser (SEQ ID NO:10);

Xaa$_{41}$ is selected from the group consisting of Lys or Arg; and
wherein R$_5$ is selected from the group consisting of NH$_2$, OH, Gly-NH$_2$, or Gly-OH.

In still other aspects, the invention provides modified forms of the GLP-1(7-34); (7-35); (7-36) or (7-37) human peptide or the C-terminal amidated forms thereof. The native peptides have the amino acid sequence (SEQ ID NO:11):

$$7 \qquad 10 \qquad\qquad 15 \qquad\qquad 20 \qquad\qquad 25$$

$$H-A-E-G-T-F-T-S-D-V-S-S-Y-L-E-G-Q-A-\dot{A}-K-E-F$$

$$30 \qquad\qquad\qquad 37$$

$$-I-A-W-L-V-K-(G)-(R)-(G)$$

wherein (G), (R), and (G) are present or absent depending on the indicated chain length. The modified forms contain one or more alterations of the native structure and are of improved ability for therapeutic use. Either the modified forms have greater potency than glucagon to potentiate insulin secretion or enhanced stability in plasma or both.

The analogs of the invention may have the foregoing sequence, or a C-terminal amide thereof, with at least one modification of SEQ ID NO:11, selected from the group consisting of:

(a) substitution of a neutral amino acid, arginine, or a D form of lysine for lysine at position 26 and/or 34 and/or a neutral amino acid, lysine, or a D form of arginine for arginine at position 36;
(b) substitution of an oxidation-resistant amino acid for tryptophan at position 31;
(c) substitution according to at least one of:

Y for V at position 16;
K for S at position 18;
D for E at position 21;
S for G at position 22;
R for Q at position 23;
R for A at position 24; and
Q for K at position 26;

(d) a substitution comprising at least one of:

an alternative small neutral amino acid for A at position 8;
an alternative acidic amino acid or neutral amino acid for E at position 9;
an alternative neutral amino acid for G at position 10; and
an alternative acidic amino acid for D at position 15; and

(e) substitution of an alternative neutral amino acid or the D or N-acylated or alkylated form of histidine for histidine at position 7.

With respect to modifications (a), (b), (d) and (e), the substituted amino acids may be in the D form, as indicated by a superscript †, e.g., C†. The amino acids substituted at position 7 can also be in the N-acylated or N-alkylated forms.

In another aspect, the invention is directed to peptides which show enhanced degradation resistance in plasma as compared to GLP-1(7-37) wherein this enhanced resistance to degradation is defined as set forth below. In these analogs, any of the above-mentioned truncated forms of GLP-1(7-34) to GLP-1(7-37) or their C-terminal amidated form is modified by

(a) substitution of a D-neutral or D-acidic amino acid for H at position 7, or
(b) substitution of a D-amino acid for A at position 8, or
(c) both, or
(d) substitution of an N-acylated or N-alkylated form of any naturally occurring amino acid for H at position 7.

Thus, analogs of the invention which are resistant to degradation include (N-acyl (1-6C) AA)[7] GLP-1(7-37) and (N-alkyl (1-6C) AA)[7] GLP-1(7-37) wherein when AA is a lysyl residue, one or both nitrogens may be alkylated or acylated. AA symbolizes any amino acid consistent with retention of insulin stimulating activity.

For substitutions of D-amino acids in the 7 and 8 positions of SEQ ID NO:11, the D residue of any acidic or neutral amino acid can be used at position 7 and ofany amino acid at position 8, again consistent with insulin stimulating activity. Either or both of position 7 and 8 can be substituted by a D-amino acid; the D-amino acid at position 7 can also be acylated or alkylated as set forth above. These modified forms are applicable not only to GLP-1(7-37) but also the shorter truncated analogs as set forth above.

Other modified GLP-1s, as well as exendins, useful in the practice of the claimed invention can be found in U.S. Patent No. 6,528,486. Further, agonists of glucagon-like peptide that exhibit activity through a GLP-1 (7-36)amide receptor have been described. See EP 0709179 A2, Hjorth et al., J. Biol. Chem. 269; 30121 (1994); Siegel et al., Amer. Diabetes Assoc, 57th Scientific Session, Boston (1997); Hareter et al., Amer. Diabetes Assoc. 57th Scientific Session, Boston (1997); Adelhorst et al., J. Biol. Client. 269, 6275 (1994); Deacon et al., 16th International Diabetes Federation Congress Abstracts, Diabetologia Supplement (1997); Irwin et al., Proc. Natl. Acad. Sci. USA 94; 7915 (1997); Mojsov, Int. J. Peptide Protein Res. 40; 333 (1992). Göke & Byrne, Diabetic Medicine 13; 854 (1996). Recent publications disclose Black Widow GLP-1 and Ser[2] GLP-1. See Holz & Hakner, Comp. Biochem. Physiol., Part B 121; 177 (1998) and Ritzel et al., J. Endocrinol 159; 93 (1998).

As previously stated, GLP-1 analogs, as well as exendin analogs, may be peptides containing one or more amino acid substitutions, additions, extensions, or deletions, compared with GLP-1(7-36), exendin-4 or exendin-3. In one embodiment, the number of substitutions, deletions, or additions is 30 amino acids or less, 25 amino acids or less, 20 amino acids or less, 15 amino acids or less, 10 amino acids or less, 5 amino acids or less or any integer in between these amounts. In one aspect of the invention, the substitutions include one or more conservative substitutions. A "conservative" substitution denotes the replacement of an amino acid residue by another, biologically active similar residue as is well known in the art Examples of conservative substitutions include the substitution of one hydrophobic residue, such as isoleucine, valine, leucine, or methionine for another, or the substitution of one polar residue for another, such as the substitution of arginine for lysine, glutamic for aspartic acids, or glutamine for asparagine, and the like.

It is further understood that GLP-1 analogs include the above described peptides which have been chemically derivatized or altered, for example, peptides with non-natural amino acid residues (*e.g.,* taurine, β- and γ-amino acid residues and D-amino acid residues), C-terminal functional group modifications, such as amides, esters, and C-terminal ketone modifications and N-terminal functional group modifications, such as acylated amines, Schiff bases, or cyclization, as found, for example, in the amino acid pyroglutamic acid. Exendin analogs, including those described below, may have similar modifications.

Other compositions of the invention include exendins, which refer to naturally occurring exendin peptides that are found in Gila-monster and related peptides. Preferred exendins include exendin-3 (SEQ ID NO: 12), which is present in the salivary secretions of *Heloderma horridum,* exendin-4 (SEQ ID NO:14), which is a peptide present in the salivary secretions of *Heloderma suspecium* (Eng., J., et al., J. Biol. Chem., 265:20259-62, 1990; Eng., J., et al., J. Biol. Chem., 267:7402-05, 1992), and agonists, analogs, derivatives, or variants of either of them, as well as biologically active fragments thereof. Exendin-4, as it occurs in the salivary secretions of the Gila monster, is an amidated peptide. However, it should be understood that the terms "exendin," "exendin-3," and "exendin-4" refer to both the amidated form of the peptide and the acid form of the peptide. Likewise, reference to GLP-1 generally refers to the amidated 7-36 molecule, but it is also intended to include non-amidated molecules, and analogs, derivatives and variants of these peptides may likewise be amidated or not

"Exendin agonist" refers to compounds that mimic any effect of an exendin by binding to a receptor or receptors where a naturally occurring exendin exerts an effect. Exendin "agonist activity" in this context means having a biological activity of an exendin, such as those described herein; but it is understood that the activity of the agonist can be either less potent or more potent than the native exendin.

Exendin-4 is a 39-amino acid polypeptide. Certain sequences of molecules of the invention are compared in Table 1.

TABLE 1

| | |
|---|---|
| a. | H A E G T F T S D V S S Y L E G Q A A K E F I A W L V K G R (NH$_2$) |
| b. | H S D G T F T S D L S K Q M E E E A V R L F I E W L K N G G P S S G A P P P S (NH$_2$) |
| c. | D L S K Q M E E E A V R L F I E W L K N G G P S S G A P P P S (NH$_2$) |
| d. | H G E G T F T S D L S K Q M E E E A V R L F I E W L K N G G P S S G A P P P S (NH$_2$) |
| e. | H S D A T F T A E Y S K L L A K L A L Q K Y L E S I L G S S T S P R P P S S |
| f. | H S D A T F T A E Y S K L L A K L A L Q K Y L E S I L G S S T S P R P P S |
| g. | H S D A I F T E E Y S K L L A K L A L Q K Y L A S I L G S R T S P P P (NH$_2$) |
| h. | H S D A I F T Q Q Y S K L L A K L A L Q K Y L A S I L G S R T S P P P (NH$_2$) |

| | |
|---|---|
| a = | GLP-1(7-36) (NH$_2$) [SEQ ID NO: 1]. |
| b = | exendin 3 (NH$_2$) [SEQ ID NO: 12]. |
| c = | exendin 4 (9-39)(NH$_2$) [SEQ ID NO: 13] (an antagonist of exendin-4 and GLP-1) |
| d = | exendin 4 (NH$_2$) [SEQ ID NO: 14]. |
| e = | helospectin I [SEQ ID NO: 15]. |
| f = | helospectin II [SEQ ID NO: 16]. |
| g = | helodermin (NH$_2$) [SEQ ID NO: 17]. |
| h = | $Q^8$, $Q^9$ helodermin (NH$_2$) [SEQ ID NO: 18]. |

Various experiments have compared the biologic actions of exendin-4 and GLP-1 and demonstrated a more favorable spectrum of properties for exendin-4 for certain indications. Exendin has been shown to lower plasma glucose, lower HbA$_{1c}$ (a measure of glycosylated hemoglobin used to evaluate plasma glucose levels), improve insulin sensitivity, and improve insulin response to glucose. Higher plasma glucose concentrations are associated with greater glucose-lowering effects, thus the observed glucose lowering effect of exendin-4 appears to be glucose-dependent, and minimal if animals are already euglycemic. Degradation studies with exendin-4 compared to GLP-1 indicate that exendin-4 is relatively resistant to degradation.

As used in this specification, the term "exendin agonist" includes any molecules, whether they be peptides, peptide mimetics, or other chemical compounds, that bind to or activate a receptor or receptors at which exendin exerts an effect, including one of those described above. Exendin agonists may include molecules having insulinotropic activity and that may bind a GLP-1 receptor molecule in *in vitro* assays and induce second messenger activity on, *inter alia*, insulin producing β-cells, but these actions are not necessary for an exendin agonist or analog to be useful in the instant invention.

The structure activity relationship (SAR) of exendin was investigated for structures that may relate to the activity of exendin, for its stability to metabolism, and for improvement of its physical characteristics, especially as it pertains to peptide stability and to amenability to alternative delivery systems, and various exendin agonist peptide compounds have been invented. Exendin agonists include exendin analogs with agonist activity in which one or more naturally or non-naturally occurring amino acids are added, inserted, eliminated or replaced with another amino acid(s). Preferred exendin analogs are peptide analogs of exendin-4.

Exendin analogs include peptides that are encoded by polynucleotides that express biologically active exendin analogs with agonist activity; and which are functional in the invention, as defined herein. For instance, exendin analogs useful in the invention may be peptides containing one or more amino acid substitutions, extensions, additions or deletions, compared with exendin-4 or exendin-3. In one embodiment, the number of substitutions, extensions, deletions, or additions is 30 amino acids or less, 25 amino acids or less, 20 amino acids or less, 15 amino acids or less, 10 amino acids or less, 5 amino acids or less or any integer in between these amounts. In one aspect of the invention, the substitutions include one or more conservative substitutions. Exendin analogs, which include chemically derivatized or altered compounds and peptides having a preferred amino acid homology to SEQ ID NOs: 12 and 14 have been previously described and are contemplated to be within the scope of the claimed invention.

Novel exendin analogs with agonist activity are described in PCT Application Serial No. PCT/US98/16387 filed August 6, 1998, entitled "Novel Exendin Agonist Compounds," which claims the benefit of U.S. Patent Application Serial No. 60/055,404, filed August 8, 1997 .

Other novel exendin analogs with agonist activity are described in PCT Application Serial No. PCT/US98/24210, filed November 13, 1998, entitled "Novel Exendin Agonist Compounds," which claims the benefit of U.S. Provisional Application No. 60/065,442 filed November 14, 1997.

Still other novel exendin analogs with agonist activity are described in PCT Application Serial No. PCT/US98/24273, filed November 13, 198, entitled "Novel Exendin Agonist. Compounds," which claims the benefit of U.S. Provisional Application No. 60/066,029 filed November 14, 1997.

Still other exendin analogs with agonist activity are described in PCT Application Serial No. PCT/US97/14199, filed August 8, 1997, entitled "Methods for Regulating Gastrointestinal Activity," which is a continuation-in-part of U.S. Patent Application Serial No. 08/694,954 filed August 8,1996.

Still other exendin analogs with agonist activity are described in PCT Application Serial No. PCT/US98/00449, filed January 7, 1998, entitled "Use of Exendins and Agonists Thereof for the Reduction of Food Intake," which claims priority to U.S. Provisional Application No. 60/034,905 filed January 7, 1997.

Exendin agonist activity can be evaluated, for example, by ascertaining activity in the assays in the referenced applications. Effects of exendins or exendin agonists can be identified, evaluated, or screened for, using the methods described therein, or other art-known or equivalent methods for determining the effects of exendin. Screening assays for potential exendin agonist compounds or candidate exendin agonist compounds, may include an in vitro GLP-1 receptor competitive assay or direct binding screen, or an activity screen, such as increased cAMP production or insulin synthesis.

Certain preferred exendin analogs with agonist activity include:

Exendin-4 (1-30) [SEQ ID NO: 19: His Gly Glu Gly Thr Phe Thr Ser Asp Leu Ser Lys Gln Met Glu Glu Glu Ala Val Arg Leu Phe Ile Glu Trp Leu Lys Asn Gly Gly];
Exendin-4 (1-30) amide [SEQ ID NO:20: His Gly Glu Gly Thr Phe Thr Ser Asp Leu Ser Lys Gln Met Glu Glu Glu Ala Val Arg Leu Phe Ile Glu Trp Leu Lys Asn Gly Gly-NH$_2$];
Exendin-4 (1-28) amide [SEQ ID NO:21: His Gly Glu Gly Thr Phe Thr Ser Asp Leu Ser Lys Gln Met Glu Glu Glu Ala Val Arg Leu Phe Ile Glu Trp Leu Lys Asn-NH$_2$];
[14]Leu,[25]Phe exendin-4 amide [SEQ ID NO:22: His Gly Gln Gly Thr Phe Thr Ser Asp Leu Ser Lys Gln Leu Glu Glu Glu Ala Val Arg Leu Phe Ile Glu Phe Leu Lys Asn Gly Gly Pro Ser Ser Gly Ala Pro Pro Pro Ser-NH$_2$];
[14]Leu, [25]Phe exendin-4 (1-28) amide [SEQ ID NO:23: His Gly Glu Gly Thr Phe Thr Ser Asp Leu Ser Lys Gln Leu Glu Glu Glu Ala Val Arg Leu Phe Ile Glu Phe Leu Lys Asn-NH$_2$]; and
[14]Leu, [22]Ala, [25]Phe exendin-4 (1-28) amide [SEQ ID NO:24: His Gly Glu Gly Thr Phe Thr Ser Asp Leu Ser Lys Gln Leu Glu Glu Glu Ala Val Arg Leu Ala Ile Glu Phe Leu Lys Asn-NH$_2$].

Also included within the scope of the present invention are pharmaceutically acceptable salts of the compounds of formula (III-X) and pharmaceutical compositions including said compounds and salts thereof.

FORMULA III

Exendin analogs with agonist activity also include those described in U.S. Serial No. 09/554,533, including compounds of the formula (III) [SEQ ID NO:25]:

Xaa$_1$ Xaa$_2$ Xaa$_3$ Gly Xaa$_5$ Xaa$_6$ Xaa$_7$ Xaa$_8$ Xaa$_9$ Xaa$_{10}$
Xaa$_{11}$ Xaa$_{12}$ Xaa$_{13}$ Xaa$_{14}$ Xaa$_{15}$ Xaa$_{16}$ Xaa$_{17}$ Ala Xaa$_{19}$ Xaa$_{20}$
Xaa$_{21}$ Xaa$_{22}$ Xaa$_{23}$ Xaa$_{24}$ Xaa$_{25}$ Xaa$_{26}$ Xaa$_{27}$ Xaa$_{28}$-Z$_1$;
wherein
Xaa$_1$ is His, Arg or Tyr;
Xaa$_2$ is Ser, Gly, Ala or Thr;
Xaa$_3$ is Asp or Glu;
Xaa$_5$ is Ala or Thr;
Xaa$_6$ is Ala, Phe, Tyr or naphthylalanine;
Xaa$_7$ is Thr or Ser;
Xaa$_8$ is Ala, Ser or Thr;
Xaa$_9$ is Asp or Glu;
Xaa$_{10}$ is Ala, Leu, Ile, Val, pentylglycine or Met;
Xaa$_{11}$ is Ala or Ser;
Xaa$_{12}$ is Ala or Lys;
Xaa$_{13}$ is Ala or Gln;
Xaa$_{14}$ is Ala, Leu, Ile, pentylglycine, Val or Met;
Xaa$_{15}$ is Ala or Glu;
Xaa$_{16}$ is Ala or Glu;
Xaa$_{17}$ is Ala or Glu;
Xaa$_{19}$ is Ala or Val;
Xaa$_{20}$ is Ala or Arg;
Xaa$_{21}$ is Ala or Leu;
Xaa$_{22}$ is Ala, Phe, Tyr or naphthylalanine;
Xaa$_{23}$ is Ile, Val, Leu, pentylglycine, tert-butylglycine or Met;
Xaa$_{24}$ is Ala, Glu or Asp;
Xaa$_{25}$ is Ala, Trp, Phe, Tyr or naphthylalanine;
Xaa$_{26}$ is Ala or Leu;
Xaa$_{27}$ is Ala or Lys;
Xaa$_{28}$ is Ala or Asn;
Z$_1$ is-OH,
-NH$_2$
Gly-Z$_2$,
Gly Gly-Z$_2$,
Gly Gly Xaa$_{31}$-Z$_2$,
Gly Gly Xaa$_{31}$ Ser-Z$_2$,
Gly Gly Xaa$_{31}$ Ser Ser-Z$_2$,
Gly Gly Xaa$_{31}$ Ser Ser Gly-Z$_2$,
Gly Gly Xaa$_{31}$ Ser Ser Gly Ala-Z$_2$,
Gly Gly Xaa$_{31}$ Ser Ser Gly Ala Xaa$_{36}$-Z$_2$,
Gly Gly Xaa$_{31}$ Ser Ser Gly Ala Xaa$_{36}$ Xaa$_{37}$-Z$_2$ or
Gly Gly Xaa$_{31}$ Ser Ser Gly Ala Xaa$_{36}$ Xaa$_{37}$ Xaa$_{38}$-Z$_2$;
Xaa$_{31}$, Xaa$_{36}$, Xaa$_{37}$ and Xaa$_{38}$ are independently Pro,homoproline, 3Hyp, 4Hyp, thioproline, N-alkylglycine, N-alkylpentylglycine or N-alkylalanine; and
Z$_2$ is -OH or -NH$_2$;

provided that no more than three of Xaa$_3$, Xaa$_5$, Xaa$_6$, Xaa$_8$, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Xaa$_{21}$, Xaa$_{24}$, Xaa$_{25}$, Xaa$_{26}$, Xaa$_{27}$ and Xaa$_{28}$ are Ala.
Preferred N-alkyl groups for N-alkylglycine, N-alkylpentylglycine and N-alkylalanine include lower alkyl groups preferably of 1 to about 6 carbon atoms, more preferably of 1 to 4 carbon atoms.

Preferred exendin analogs include those wherein $Xaa_1$ is His or Tyr. More preferably $Xaa_1$ is His.

Preferred are those compounds wherein $Xaa_2$ is Gly.

Preferred are those compounds wherein $Xaa_{14}$ is Leu, pentylglycine or Met.

Preferred compounds are those wherein $Xaa_{25}$ is Trp or Phe.

Preferred compounds are those where $Xaa_6$ is Phe or naphthylalanine; $Xaa_{22}$ is Phe or naphthylalanine and $Xaa_{23}$ is Ile or Val.

Preferred are compounds wherein $Xaa_{31}$, $Xaa_{36}$, $Xaa_{37}$ and $Xaa_{38}$ are independently selected from Pro, homoproline, thioproline and N-alkylalanine.

Preferably $Z_1$ is $-NH_2$.

Preferably $Z_2$ is $-NH_2$.

According to one aspect, preferred are compounds of formula (III) wherein $Xaa_1$ is His or Tyr, more preferably His; $Xaa_2$ is Gly; $Xaa_6$ is Phe or naphthylalanine; $Xaa_{14}$ is Leu, pentylglycine or Met; $Xaa_{22}$ is Phe or naphthylalanine; $Xaa_{23}$ is Ile or Val; $Xaa_{31}$, $Xaa_{36}$, $Xaa_{37}$ and $Xaa_{38}$ are independently selected from Pro, homoproline, thioproline or N-alkylalanine. More preferably $Z_1$ is $-NH_2$.

According to an especially preferred aspect, especially preferred compounds include those of formula (III) wherein: $Xaa_1$ is His or Arg; $Xaa_2$ is Gly or Ala; $Xaa_3$ is Asp or Glu; $Xaa_5$ is Ala or Thr; $Xaa_6$ is Ala, Phe or nephthylalaine; $Xaa_7$ is Thr or Ser; $Xaa_8$ is Ala, Ser or Thr; $Xaa_9$ is Asp or Glu; $Xaa_{10}$ is Ala, Leu or pentylglycine; $Xaa_{11}$ is Ala or Ser; $Xaa_{12}$ is Ala or Lys; $Xaa_{13}$ is Ala or Gln; $Xaa_{14}$ is Ala, Leu or pentylglycine; $Xaa_{15}$ is Ala or Glu; $Xaa_{16}$ is Ala or Glu; $Xaa_{17}$ is Ala or Glu; $Xaa_{19}$ is Ala or Val; $Xaa_{20}$ is Ala or Arg; $Xaa_{21}$ is Ala or Leu; $Xaa_{22}$ is Phe or naphthylalanine; $Xaa_{23}$ is Ile, Val or tert-butylglycine; $Xaa_{24}$ is Ala, Glu or Asp; $Xaa_{25}$ is Ala, Trp or Phe; $Xaa_{26}$ is Ala or Leu; $Xaa_{27}$ is Ala or Lys; $Xaa_{28}$ is Ala or Asn; $Z_1$ is $-OH$, $-NH_2$, Gly-$Z_2$, Gly Gly-$Z_2$, Gly Gly $Xaa_{31}$-$Z_2$, Gly Gly $Xaa_{31}$ Ser-$Z_2$, Gly Gly $Xaa_{31}$ Ser Ser-$Z_2$, Gly Gly $Xaa_{31}$ Ser Ser Gly-$Z_2$, Gly Gly $Xaa_{31}$ Ser Ser Gly Ala-$Z_2$, Gly Gly $Xaa_{31}$ Ser Ser Gly Ala $Xaa_{36}$-$Z_2$, Gly Gly $Xaa_{31}$ Ser Ser Gly Ala $Xaa_{36}$ $Xaa_{37}$-$Z_2$, Gly Gly $Xaa_{31}$ Ser Ser Gly Ala $Xaa_{36}$ $Xaa_{37}$ $Xaa_{38}$-$Z_2$; $Xaa_{31}$, $Xaa_{36}$, $Xaa_{37}$ and $Xaa_{38}$ being independently Pro homoproline, thioproline or N-methylalanine; and $Z_2$ being $-OH$ or $-NH_2$; provided that no more than three of $Xaa_3$, $Xaa_5$, $Xaa_6$, $Xaa_8$, $Xaa_{10}$, $Xaa_{11}$, $Xaa_{12}$, $Xaa_{13}$, $Xaa_{14}$, $Xaa_{15}$, $Xaa_{16}$, $Xaa_{17}$, $Xaa_{19}$, $Xaa_{20}$, $Xaa_{21}$, $Xaa_{24}$, $Xaa_{25}$, $Xaa_{26}$, $Xaa_{27}$ and $Xaa_{28}$ are Ala. Especially preferred compounds include those set forth in PCT application Serial No. PCT/US98/24210, filed November 13, 1998, entitled "Novel Exendin Agonist Compounds" identified therein as compounds 2-23.

According to an especially preferred aspect, provided are compounds where $Xaa_{14}$ is Leu, Ile, Val or pentylglycine, more preferably Leu or pentylglycine, and $Xaa_{25}$ is Phe, Tyr or naphthylalanine, more preferably Phe or naphthylalanine. These compounds will be less susceptive to oxidative degration, both in vitro and in vivo, as well as during synthesis of the compound.

FORMULA IV

Exendin analogs with agonist activity also include those described in U.S. Provisional Application No. 09/554,531, including compounds of the formula (IV)[SEQ ID NO:26]:

$Xaa_1$ $Xaa_2$ $Xaa_3$ $Xaa_4$ $Xaa_5$ $Xaa_6$ $Xaa_7$ $Xaa_8$ $Xaa_9$ $Xaa_{10}$
$Xaa_{11}$ $Xaa_{12}$ $Xaa_{13}$ $Xaa_{14}$ $Xaa_{15}$ $Xaa_{16}$ $Xaa_{17}$ Ala $Xaa_{19}$ $Xaa_{20}$
$Xaa_{21}$ $Xaa_{22}$ $Xaa_{23}$ $Xaa_{24}$ $Xaa_{25}$ $Xaa_{26}$ $Xaa_{27}$ $Xaa_{28}$-$Z_1$;
wherein:
$Xaa_1$ is His, Arg, Tyr, Ala, Norval, Val or Norleu;
$Xaa_2$ is Ser, Gly, Ala or Thr;
$Xaa_3$ is Ala, Asp or Glu;
$Xaa_4$ is Ala, Norval, Val, Norleu or Gly;
$Xaa_5$ is Ala or Thr;
$Xaa_6$ is Phe, Tyr or naphthylalanine;
$Xaa_7$ is Thr or Ser;
$Xaa_8$ is Ala, Ser or Thr;
$Xaa_9$ is Ala, Norval, Val, Norleu, Asp or Glu;
$Xaa_{10}$ is Ala, Leu, Ile, Val, pentylglycine or Met;
$Xaa_{11}$ is Ala or Ser;
$Xaa_{12}$ is Ala or Lys;
$Xaa_{13}$ is Ala or Gln;
$Xaa_{14}$ is Ala, Leu, Ile, pentylglycine, Val or Met;
$Xaa_{15}$ is Ala or Glu;
$Xaa_{16}$ is Ala or Glu;

Xaa$_{17}$ is Ala or Glu;
Xaa$_{19}$ is Ala or Val;
Xaa$_{20}$ is Ala or Arg;
Xaa$_{21}$ is Ala or Leu;
Xaa$_{22}$ is Phe, Tyr or naphthylalanine;
Xaa$_{23}$ is Ile, Val, Leu, pentylglycine, tert-butylglycine or Met;
Xaa$_{24}$ is Ala, Glu or Asp;
Xaa$_{25}$ is Ala, Trp, Phe, Tyr or naphthylalanine;
Xaa$_{26}$ is Ala or Leu;
Xaa$_{27}$ is Ala or Lys;
Xaa$_{28}$ is Ala or Asn;
Z$_1$ is -OH,

-NH$_2$,
Gly-Z$_2$,
Gly Gly-Z$_2$,
Gly Gly Xaa$_{31}$-Z$_2$,
Gly Gly Xaa$_{31}$ Ser-Z$_2$,
Gly Gly Xaa$_{31}$ Ser Ser-Z$_2$,
Gly Gly Xaa$_{31}$ Ser Ser Gly-Z$_2$,
Gly Gly Xaa$_{31}$ Ser Ser Gly Ala-Z$_2$,
Gly Gly Xaa$_{31}$ Ser Ser Gly Ala Xaa$_{36}$-Z$_2$,
Gly Gly Xaa$_{31}$ Ser Ser Gly Ala Xaa$_{36}$ Xaa$_{37}$-Z$_2$,
Gly Gly Xaa$_{31}$ Ser Ser Gly Ala Xaa$_{36}$ Xaa$_{37}$ Xaa$_{38}$-Z$_2$ or
Gly Gly Xaa$_{31}$ Ser Ser Gly Ala Xaa$_{36}$ Xaa$_{37}$ Xaa$_{38}$ Xaa$_{39}$-Z$_2$;
Xaa$_{31}$, Xaa$_{36}$, Xaa$_{37}$ and Xaa$_{38}$ are independently Pro, homoproline, 3Hyp, 4Hyp, thioproline, N-alkylglycine, N-alkylpentylglycine or N-alkylalanine; and
Z$_2$ is -OH or -NH$_2$;

provided that no more than three of Xaa$_3$, Xaa$_4$, Xaa$_5$, Xaa$_6$, Xaa$_8$, Xaa$_9$, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Xaa$_{21}$, Xaa$_{24}$, Xaa$_{25}$, Xaa$_{26}$, Xaa$_{27}$ and Xaa$_{28}$ are Ala; and provided also that, if Xaa$_1$ is His, Arg or Tyr, then at least one of Xaa$_3$, Xaa$_4$ and Xaa$_9$ is Ala.

Preferred N-alkyl groups for N-alkylglycine, N-alkylpentylglycine and N-alkylalanine include lower alkyl groups preferably of 1 to about 6 carbon atoms, more preferably of 1 to 4 carbon atoms. Suitable compounds of formula (II) include those described in application Serial No. PCT/US98/24273, filed November 13, 1998, entitled "Novel Exendin Agonist Compounds," identified therein in Examples 1-89 ("Compounds 1-89," respectively), as well as those corresponding compounds identified therein in Examples 104 and 105.

Preferred such exendin analogs include those wherein Xaa$_1$ is His, Ala or Norval. More preferably Xaa$_1$ is His or Ala. Most preferably Xaa$_1$ is His.

Preferred are those compounds of formula (IV) wherein Xaa$_2$ is Gly.

Preferred are those compounds of formula (TV) wherein Xaa$_3$ is Ala.

Preferred are those compounds of formula (IV) wherein Xaa$_4$ is Ala.

Preferred are those compounds of formula (IV) wherein Xaa$_9$ is Ala.

Preferred are those compounds of formula (IV) wherein Xaa$_{14}$ is Leu, pentylglycine or Met.

Preferred compounds of formula (IV) are those wherein Xaa$_{25}$ is Trp or Phe.

Preferred compounds of formula (IV) are those where Xaa$_6$ is Ala, Phe or naphthylalanine; Xaa$_{22}$ is Phe or naphthylalanine; and Xaa$_{23}$ is Ile or Val.

Preferred are compounds of formula (IV) wherein Xaa$_{31}$, Xaa$_{36}$, Xaa$_{37}$ and Xaa$_{38}$ are independently selected from Pro, homoproline, thioproline and N-alkylalanine.

Preferably Z$_1$ is -NH$_2$.

Preferably Z$_2$ is -NH$_2$.

According to one aspect, preferred are compounds of formula (TV) wherein Xaa$_1$ is Ala, His or Tyr, more preferably Ala or His; Xaa$_2$ is Ala or Gly; Xaa$_6$ is Phe or naphthylalanine; Xaa$_{14}$ is Ala, Leu, pentylglycine or Met; Xaa$_{22}$ is Phe or naphthylalanine; Xaa$_{23}$ is Ile or Val; Xaa$_{31}$, Xaa$_{36}$, Xaa$_{37}$ and Xaa$_{38}$ are independently selected from Pro, homoproline, thioproline or N-alkylalanine; and Xaa$_{39}$ is Ser or Tyr, more preferably Ser. More preferably Z$_1$ is -NH$_2$.

In an especially preferred aspect, preferred compounds include those of formula (IV) wherein: Xaa$_1$ is His or Ala; Xaa$_2$ is Gly or Ala; Xaa$_3$ is Ala, Asp or Glu; Xaa$_4$ is Ala or Gly; Xaa$_5$ is Ala or Thr; Xaa$_6$ is Phe or naphthylalanine; Xaa$_7$ is Thr or Ser; Xaa$_8$ is Ala, Ser or Thr; Xaa$_9$ is Ala, Asp or Glu; Xaa$_{10}$ is Ala, Leu or pentylglycine; Xaa$_{11}$ is Ala or

Ser; Xaa$_{12}$ is Ala or Lys; Xaa$_{13}$ is Ala or Gln; Xaa$_{14}$ is Ala, Leu, Met or pentylglycine; Xaa$_{15}$ is Ala or Glu; Xaa$_{16}$ is Ala or Glu; Xaa$_{17}$ is Ala or Glu; Xaa$_{19}$ is Ala or Val; Xaa$_{20}$ is Ala or Arg; Xaa$_{21}$ is Ala or Leu; Xaa$_{22}$ is Phe or naphthylalanine; Xaa$_{23}$ is Ile, Val or tert-butylglycine; Xaa$_{24}$ is Ala, Glu or Asp; Xaa$_{25}$ is Ala, Trp or Phe; Xaa$_{26}$ is Ala or Leu; Xaa$_{27}$ is Ala or Lys; Xaa$_{28}$ is Ala or Asn; Z$_1$ is - OH, -NH$_2$, Gly-Z$_2$, Gly Gly-Z$_2$, Gly Gly Xaa$_{31}$-Z$_2$, Gly Gly Xaa$_{31}$ Ser-Z$_2$, Gly Gly Xaa$_{31}$ Ser Ser-Z$_2$, Gly Gly Xaa$_{31}$ Ser Ser Gly-Z$_2$, Gly Gly Xaa$_{31}$ Ser Ser Gly Ala-Z$_2$, Gly Gly Xaa$_{31}$ Ser Ser Gly Ala Xaa$_{36}$-Z$_2$, Gly Gly Xaa$_{31}$ Ser Ser Gly Ala Xaa$_{36}$ Xaa$_{37}$-Z$_2$, Gly Gly Xaa$_{31}$ Ser Ser Gly Ala Xaa$_{36}$ Xaa$_{37}$ Xaa$_{38}$-Z$_2$ or Gly Gly Xaa$_{31}$ Ser Ser Gly Ala Xaa$_{36}$ Xaa$_{37}$ Xaa$_{38}$ Xaa$_{39}$-Z$_2$; Xaa$_{31}$, Xaa$_{36}$, Xaa$_{37}$ and Xaa$_{38}$ being independently Pro homoproline, thioproline or N-methylalanine; and Z$_2$ being -OH or -NH$_2$; provided that no more than three of Xaa$_3$, Xaa$_5$, Xaa$_6$, Xaas, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Xaa$_{21}$, Xaa$_{24}$, Xaa$_{25}$, Xaa$_{26}$, Xaa$_{27}$ and Xaa$_{28}$ are Ala; and provided also that, if Xaa$_1$ is His, Arg or Tyr, then at least one of Xaa$_3$, Xaa$_4$ and Xaa$_9$ is Ala. Especially preferred compounds of formula (IV) include those described in application Serial No. PCT/US98/24273, filed November 13, 1998, entitled "Novel Exendin Agonist Compounds" as having the amino acid sequence of SEQ. ID. NOS. 5-93 therein.

According to an especially preferred aspect, provided are compounds of formula (IV) where Xaa$_{14}$ is Ala, Leu, Ile, Val or pentylglycine, more preferably Leu or pentylglycine, and Xaa$_{25}$ is Ala, Phe, Tyr or naphthylalanine, more preferably Phe or naphthylalanine. These compounds will be less susceptible to oxidative degration, both in vitro and in vivo, as well as during synthesis of the compound.

## FORMULA V

Also within the scope of the present invention are narrower genera of compounds having peptides of various lengths, for example genera of compounds which do not include peptides having a length of 28, 29 or 30 amino acid residues, respectively. Additionally, the present invention includes narrower genera of compounds described in PCT application Serial No. PCT/US98/24210, filed November 13, 1998, entitled "Novel Exendin Agonist Compounds" and having particular amino acid sequences, for example, compounds of the formula (V) [SEQ. ID. NO:27]:

Xaa$_1$ Xaa$_2$ Xaa$_3$ Gly Xaa$_5$ Xaa$_6$ Xaa$_7$ Xaa$_8$ Xaa$_9$ Xaa$_{10}$
Xaa$_{11}$ Xaa$_{12}$ Xaa$_{13}$ Xaa$_{14}$ Xaa$_{15}$ Xaa$_{16}$ Xaa$_{17}$ Ala Xaa$_{19}$
Xaa$_{20}$ Xaa$_{21}$ Xaa$_{22}$ Xaa$_{23}$ Xaa$_{24}$ Xaa$_{25}$ Xaa$_{26}$ Xaa$_{27}$ Xaa$_{28}$-Z$_1$;
wherein:
Xaa$_1$ is His or Arg;
Xaa$_2$ is Gly or Ala;
Xaa$_3$ is Asp or Glu;
Xaa$_5$ is Ala or Thr;
Xaa$_6$ is Ala, Phe or naphthylalanine;
Xaa$_7$ is Thr or Ser;
Xaa$_8$ is Ala, Ser or Thr;
Xaa$_9$ is Asp or Glu;
Xaa$_{10}$ is Ala, Leu or pentylglycine;
Xaa$_{11}$ is Ala or Ser;
Xaa$_{12}$ is Ala or Lys;
Xaa$_{13}$ is Ala or Gln;
Xaa$_{14}$ is Ala, Leu or pentylglycine;
Xaa$_{15}$ is Ala or Glu;
Xaa$_{16}$ is Ala or Glu;
Xaa$_{17}$ is Ala or Glu;
Xaa$_{19}$ is Ala or Val;
Xaa$_{20}$ is Ala or Arg;
Xaa$_{21}$ is Ala or Leu;
Xaa$_{22}$ is Phe or naphthylalanine;
Xaa$_{23}$ is Ile, Val or tert-butylglycine;
Xaa$_{24}$ is Ala, Glu or Asp;
Xaa$_{25}$ is Ala, Trp, or Phe;
Xaa$_{26}$ is Ala or Leu;
Xaa$_{27}$ is Ala or Lys;
Xaa$_{28}$ is Ala or Asn;
Z$_1$ is -OH,

-NH$_2$,

Gly-$Z_2$,
Gly Gly -$Z_2$,
Gly Gly Xaa$_{31}$-$Z_2$,
Gly Gly Xaa$_{31}$ Ser-$Z_2$,
Gly Gly Xaa$_{31}$ Ser Ser-$Z_2$,
Gly Gly Xaa$_{31}$ Ser Ser Gly-$Z_2$,
Gly Gly Xaa$_{31}$ Ser Ser Gly Ala-$Z_2$,
Gly Gly Xaa$_{31}$ Ser Ser Gly Ala Xaa$_{36}$-$Z_2$,
Gly Gly Xaa$_{31}$ Ser Ser Gly Ala Xaa$_{36}$ Xaa$_{37}$-$Z_2$ or
Gly Gly Xaa$_{31}$ Ser Ser Gly Ala Xaa$_{36}$ Xaa$_{37}$ Xaa$_{38}$-$Z_2$;
Xaa$_{31}$, Xaa$_{36}$, Xaa$_{37}$ and Xaa$_{38}$ are independently selected from the group consisting of Pro, homoproline, thioproline and N-methylylalanine; and
$Z_2$ is -OH or -NH$_2$;

provided that no more than three of Xaa$_3$, Xaa$_5$, Xaa$_6$, Xaa$_8$, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Baa$_{21}$, Xaa$_{24}$, Xaa$_{25}$, Xaa$_{26}$, Xaa$_{27}$ and Xaa$_{28}$ are Ala; and pharmaceutically acceptable salts thereof.

<u>FORMULA VI</u>

Additionally, the present invention includes narrower genera of peptide compounds described in PCT Application Serial No. PCT/US98/24273, filed November 13, 1998, entitled "Novel Exendin Agonist Compounds" as having particular amino acid sequences, for example, compounds of the formula [VI] [SEQ. ID. NO:28]:

Xaa$_1$ Xaa$_2$ Xaa$_3$ Xaa$_5$ Xaa$_5$ Xaa$_6$ Xaa$_7$ Xaa$_8$ Xaa$_9$ Xaa$_{10}$
Xaa$_{11}$ Xaa$_{12}$ Xaa$_{13}$ Xaa$_{14}$ Xaa$_{15}$ Xaa$_{16}$ Xaa$_{17}$ Ala Xaa$_{19}$
Xaa$_{20}$ Xaa$_{21}$ Xaa$_{22}$ Xaa$_{23}$ Xaa$_{24}$ Xaa$_{25}$ Xaa$_{26}$ Xaa$_{27}$ Xaa$_{28}$-$Z_1$;
wherein:
Xaa$_1$ is His or Ala;
Xaa$_2$ is Gly or Ala;
Xaa$_3$ is Ala, Asp or Glu;
Xaa$_4$ is Ala or Gly;
Xaa$_5$ is Ala or Thr;
Xaa$_6$ is Phe or naphthylalanine;
Xaa$_7$ is Thr or Ser;
Xaa$_8$ is Ala, Ser or Thr;
Xaa$_9$ is Ala, Asp or Glu;
Xaa$_{10}$ is Ala, Leu or pentylglycine;
Xaa$_{11}$ is Ala or Ser;
Xaa$_{12}$ is Ala or Lys;
Xaa$_{13}$ is Ala or Gln;
Xaa$_{14}$ is Ala, Leu, Met or pentylglycine;
Xaa$_{15}$ is Ala or Glu;
Xaa$_{16}$ is Ala or Glu;
Xaa$_{17}$ is Ala or Glu;
Xaa$_{19}$ is Ala or Val;
Xaa$_{20}$ is Ala or Arg;
Xaa$_{21}$ is Ala or Leu;
Xaa$_{22}$ is Phe or naphthylalanine;
Xaa$_{23}$ is Ile, Val or tert-butylglycine;
Xaa$_{24}$ is Ala, Glu or Asp;
Xaa$_{25}$ is Ala, Trp or Phe;
Xaa$_{26}$ is Ala or Leu;
Xaa$_{27}$ is Ala or Lys;
Xaa$_{28}$ is Ala or Asn;
$Z_1$ is -OH,

-NH$_2$,
Gly-$Z_2$,

Gly Gly-Z$_2$
Gly Gly Xaa$_{31}$-Z$_2$,
Gly Gly Xaa$_{31}$ Ser-Z$_2$,
Gly Gly Xaa$_{31}$ Ser Ser-Z$_2$,
Gly Gly Xaa$_{31}$ Ser Ser Gly-Z$_2$,
Gly Gly Xaa$_{31}$ Ser Ser Gly Ala-Z$_2$,
Gly Gly Xaa$_{31}$ Ser Ser Gly Ala Xaa$_{36}$-Z$_2$,
Gly Gly Xaa$_{31}$ Ser Ser Gly Ala Xaa$_{36}$ Xaa$_{37}$-Z$_2$
Gly Gly Xaa$_{31}$ Ser Ser Gly Ala Xaa$_{36}$ Xaa$_{37}$ Xaa$_{38}$-Z$_2$
Gly Gly Xaa$_{31}$ Ser Ser Gly Ala Xaa$_{36}$ Xaa$_{37}$ Xaa$_{38}$ Ser-Z$_2$;
Xaa$_{31}$, Xaa$_{36}$, Xaa$_{37}$ and Xaa$_{38}$ are independently Pro,homoproline, thioproline, or N-methylylalanine; and
Z$_2$ is -OH or -NH$_2$;

provided that no more than three of Xaa$_3$, Xaa$_5$, Xaa$_6$, Xaa$_8$, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Xaa$_{21}$, Xaa$_{24}$, Xaa$_{25}$, Xaa$_{26}$, Xaa$_{27}$, and Xaa$_{28}$ are Ala; and provided that, if Xaa$_1$ is His, Arg or Tyr, then at least one of Xaa$_3$, Xaa$_4$ and Xaa$_9$ is Ala; and pharmaceutically acceptable salts thereof.

Preferred compounds of formula (VI) include those wherein Xaa$_1$ is His, Ala, Norval or 4-imidazopropionyl. Preferably, Xaa$_1$ is His, or 4-imidazopropionyl or Ala, more preferably His or 4-imidazopropionyl.

Preferred compounds of formula (VI) include those wherein Xaa2 is Gly.

Preferred compounds of formula (VI) include those wherein Xaa4 is Ala.

Preferred compounds of formula (VI) include those wherein Xaa9 is Ala.

Preferred compounds of formula (VI) include those wherein Xaa14 is Leu, pentylglycine or Met.

Preferred compounds of formula (VI) include those wherein Xaa25 is Trp or Phe.

Preferred compounds of formula (VI) include those wherein Xaa6 is Ala, Phe or naphthylalanine; Xaa22 is Phe or naphthylalanine; and Xaa23 is Ile or Val.

Preferred compounds of formula (VI) include those wherein Z1 is -NH2.

Preferred compounds of formula (VI) include those wherein Xaa31, Xaa36, Xaa37 and Xaa38 are independently selected from the group consisting of Pro, homoproline, thioproline and N-alkylalanine.

Preferred compounds of formula (VI) include those wherein Xaa39 is Ser or Tyr, preferably Ser.

Preferred compounds of formula (VI) include those wherein Z2 is -NH2.

Preferred compounds of formula (VI) include those 42 wherein Z1 is -NH2.

Preferred compounds of formula (VI) include those wherein Xaa21 is Lys-NH2-R where R is Lys, Arg, C1-C10 straight chain or branched alkanoyl.

Preferred compounds of formula (VI) include those wherein X1 is Lys Asn, Lys-NH$\epsilon$-R Asn, or Lys-NH$\epsilon$-R Ala where R is Lys, Arg, C1-C10 straight chain or branched alkanoyl. Preferred compounds of formula (VI) include those having an amino acid sequence described in PCT application Serial No. PCT/US98/24273, filed November 13, 1998, entitled "Novel Exendin Agonist Compounds" as being selected from SEQ. ID. NOS. 95-110 therein.

FORMULA VII

Also provided are compounds described in PCT application PCT/US98/24210, filed November 13, 1998, entitled "Novel Exendin Agonist Compounds", including compounds of the formula (VII) [SEQ. ID. NO. 29]:

Xaa$_1$ Xaa$_2$ Xaa$_3$ Gly Xaa$_5$ Xaa$_6$ Xaa$_7$ Xaa$_8$ Xaa$_9$ Xaa$_{10}$
Xaa$_{11}$ Xaa$_{12}$ Xaa$_{13}$ Xaa$_{14}$ Xaa$_{15}$ Xaa$_{16}$ Xaa$_{17}$ Ala Xaa$_{19}$ Xaa$_{20}$
Xaa$_{21}$ Xaa$_{22}$ Xaa$_{23}$ Xaa$_{24}$ Xaa$_{25}$ Xaa$_{26}$ X$_1$ -Z$_1$;
wherein
Xaa$_1$ is His, Arg or Tyr or 4-imidazopropionyl;
Xaa$_2$ is Ser, Gly, Ala or Thr;
Xaa$_3$ is Asp or Glu;
Xaa$_5$ is Ala or Thr;
Xaa$_6$ is Ala, Phe, Tyr or naphthylalanine;
Xaa$_7$ is Thr or Ser;
Xaa$_8$ is Ala, Ser or Thr;
Xaa$_9$ is Asp or Glu;
Xaa$_{10}$ is Ala, Leu, Ile, Val, pentylglycine or Met;
Xaa$_{11}$ is Ala or Ser;
Xaa$_{12}$ is Ala or Lys;

Xaa$_{13}$ is Ala or Gln;
Xaa$_{14}$ is Ala, Leu, Ile, pentylglycine, Val or Met;
Xaa$_{15}$ is Ala or Glu;
Xaa$_{16}$ is Ala or Glu;
Xaa$_{17}$ is Ala or Glu;
Xaa$_{19}$ is Ala or Val;
Xaa$_{20}$ is Ala or Arg;
Xaa$_{21}$ is Ala, Leu or Lys-NH$^{\varepsilon}$-R where R is Lys, Arg, C$_1$-C$_{10}$ straight chain or branched alkanoyl or cycloalkylalkanoyl;
Xaa$_{22}$ is Phe, Tyr or naphthylalanine;
Xaa$_{23}$ is Ile, Val, Leu, pentylglycine, tert-butylglycine or Met;
Xaa$_{24}$ is Ala, Glu or Asp;
Xaa$_{25}$ is Ala, Trp, Phe, Tyr or naphthylalanine;
Xaa$_{26}$ is Ala or Leu;
X$_1$ is Lys Asn, Asn Lys, Lys-NH$^{\varepsilon}$-R Asn, Asn Lys-NH$^{\varepsilon}$-R, Lys-NH$^{\varepsilon}$-R Ala, Ala Lys-NH$^{\varepsilon}$-R where R is Lys, Arg, C$_1$-C$_{10}$ straight chain or branched alkanoyl or cycloalkylalkanoyl
Z$_1$ is -OH,

-NH$_2$,.
Gly-Z$_2$,
Gly Gly-Z$_2$,
Gly Gly Xaa$_{31}$-Z$_2$,
Gly Gly Xaa$_{31}$ Ser-Z$_2$,
Gly Gly Xaa$_{31}$ Ser Ser-Z$_2$,
Gly Gly Xaa$_{31}$ Ser Ser Gly-Z$_2$,
Gly Gly Xaa$_{31}$ Ser Ser Gly Ala-Z$_2$,
Gly Gly Xaa$_{31}$ Ser Ser Gly Ala Xaa$_{36}$-Z$_2$,
Gly Gly Xaa$_{31}$ Ser Ser Gly Ala Xaa$_{36}$ Xaa$_{37}$-Z$_2$ or
Gly Gly Xaa$_{31}$ Ser Ser Gly Ala Xaa$_{36}$ Xaa$_{37}$ Xaa$_{38}$-Z$_2$;
Xaa$_{31}$, Xaa$_{36}$, Xaa$_{37}$ and Xaa$_{38}$ are independently selected from the group consisting of Pro, homoproline, 3Hyp, 4Hyp, thioproline, N-alkylglycine, N-alkylpentylglycine and N-alkylalanine; and
Z$_2$ is -OH or -NH$_2$;

provided that no more than three of Xaa$_3$, Xaa$_5$, Xaa$_6$, Xaa$_8$, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Xaa$_{21}$, Xaa$_{24}$, Xaa$_{25}$, and Xaa$_{26}$ are Ala. Also within the scope of the present invention are pharmaceutically acceptable salts of the compound of formula (VII) and pharmaceutical compositions including said compounds and salts thereof.

Preferred exendin analogs of formula (VII) include those wherein Xaa$_1$ is His, Tyr or 4-imidazopropionyl. More preferably Xaa$_1$ is His.

Preferred are those compounds of formula (VII) wherein Xaa$_1$ is 4-imidazopropionyl. Preferred are those compounds of formula (VII) wherein Xaa$_2$ is Gly.

Preferred compounds of formula (VII) are those wherein Xaa$_{14}$ is Leu, pentylglycine or Met.

Preferred compounds of formula (VII) are those wherein Xaa$_{25}$ is Trp or Phe.

According to one aspect, preferred are compounds of formula (VII) wherein Xaa$_6$ is Phe or naphthylalanine; and Xaa$_{22}$ is Phe or naphthylalanine; and Xaa$_{23}$ is Ile or Val. More preferably, Z$_1$ is -NH$_2$. According to one aspect, especially preferred are such compounds of formula (VII) wherein Xaa$_{31}$, Xaa$_{36}$, Xaa$_{37}$ and Xaa$_{38}$ are independently selected from the group consisting of Pro, homoproline, thioproline and N-alkylalanine. More preferds, Z$_2$ is -NH$_2$.

Preferred compounds of formula (VII) include those wherein X$_1$ is Lys Asn, Lys-NH$^{\varepsilon}$-R Asn, or Lys-NH$^{\varepsilon}$-R Ala where R is Lys, Arg, C$_1$-C$_{10}$ straight chain or branched alkanoyl. Preferred compounds of formula (VII) include compounds described in PCT application Serial No. PCT/US98/24210, filed November 13, 1998, entitled "Novel Exendin Agonist Compounds" and identified therein as Compound Nos. 62-69.

Preferred such exendin analogs include those wherein Xaa$_1$ is His, Ala or Norval. More preferably Xaa$_1$ is His or Ala. Most preferably Xaa$_1$ is His.

Preferred are those compounds of formula (VII) wherein Xaa$_2$ is Gly.

Preferred are those compounds of formula (VII) wherein Xaa$_3$ is Ala.

Preferred are those compounds of formula (VII) wherein Xaa$_4$ is Ala.

Preferred are those compounds of formula (VII) wherein Xaa$_9$ is Ala.

Preferred are those compounds of formula (VII) wherein Xaa$_{14}$ is Leu, pentylglycine or Met.

Preferred compounds of formula (VII) are those wherein Xaa$_{25}$ is Trp or Phe.

Preferred compounds of formula (VII) are those where $Xaa_6$ is Ala, Phe or naphthylalanine; $Xaa_{22}$ is Phe or naphthylalanine; and $Xaa_{23}$ is Ile or Val.

Preferred are compounds of formula (VII) wherein $Xaa_{31}$, $Xaa_{36}$, $Xaa_{37}$ and $Xaa_{38}$ are independently selected from Pro, homoproline, thioproline and N-alkylalanine.

Preferably $Z_1$ is $-NH_2$.

Preferably $Z_2$ is $-NH_2$.

According to one aspect, preferred are compounds of formula (VII) wherein $Xaa_1$ is Ala, His or Tyr, more preferably Ala or His; $Xaa_2$ is Ala or Gly; $Xaa_6$ is Phe or naphthylalanine; $Xaa_{14}$ is Ala, Leu, pentylglycine or Met; $Xaa_{22}$ is Phe or naphthylalanine; $Xaa_{23}$ is Ile or Val; $Xaa_{31}$, $Xaa_{36}$, $Xaa_{37}$ and $Xaa_{38}$ are independently selected from Pro, homoproline, thioproline or N-alkylalanine; and $Xaa_{39}$ is Ser or Tyr, more preferably Ser. More preferably $Z_1$ is $-NH_2$.

According to an especially preferred aspect, preferred compounds include those of formula (VII) wherein: $Xaa_1$ is His or Ala; $Xaa_2$ is Gly or Ala; $Xaa_3$ is Ala, Asp or Glu; $Xaa_4$ is Ala or Gly; $Xaa_5$ is Ala or Thr; $Xaa_6$ is Phe or naphthylalanine; $Xaa_7$ is Thr or Ser; $Xaa_8$ is Ala, Ser or Thr; $Xaa_9$ is Ala, Asp or Glu; $Xaa_{10}$ is Ala, Leu or pentylglycine; $Xaa_{11}$ is Ala or Ser; $Xaa_{12}$ is Ala or Lys; $Xaa_{13}$ is Ala or Gln; $Xaa_{14}$ is Ala, Leu, Met or pentylglycine; $Xaa_{15}$ is Ala or Glu; $Xaa_{16}$ is Ala or Glu; $Xaa_{17}$ is Ala or Glu; $Xaa_{19}$ is Ala or Val; $Xaa_{20}$ is Ala or Arg; $Xaa_{21}$ is Ala or Leu; $Xaa_{22}$ is Phe or naphthylalanine; $Xaa_{23}$ is Ile, Val or tert-butylglycine; $Xaa_{24}$ is Ala, Glu or Asp; $Xaa_{25}$ is Ala, Trp or Phe; $Xaa_{26}$ is Ala or Leu; $Xaa_{27}$ is Ala or Lys; $Xaa_{28}$ is Ala or Asn; $Z_1$ is -OH, $-NH_2$, Gly-$Z_2$, Gly Gly-$Z_2$, Gly Gly $Xaa_{31}$-$Z_2$, Gly Gly $Xaa_{31}$ Ser-$Z_2$, Gly Gly $Xaa_{31}$ Ser Ser-$Z_2$, Gly Gly $Xaa_{31}$ Ser Ser Gly-$Z_2$, Gly Gly $Xaa_{31}$ Ser Ser Gly Ala-$Z_2$, Gly Gly $Xaa_{31}$ Ser Ser Gly Ala $Xaa_{36}$-$Z_2$, Gly Gly $Xaa_{31}$ Ser Ser Gly Ala $Xaa_{36}$ $Xaa_{37}$-$Z_2$, Gly Gly $Xaa_{31}$ Ser Ser Gly Ala $Xaa_{36}$ $Xaa_{37}$ $Xaa_{38}$-$Z_2$ or Gly Gly $Xaa_{31}$ Ser Ser Gly Ala $Xaa_{36}$ $Xaa_{37}$ $Xaa_{38}$ $Xaa_{39}$-$Z_2$; $Xaa_{31}$, $Xaa_{36}$, $Xaa_{37}$ and $Xaa_{38}$ being independently Pro homoproline, thioproline or N-methylalanine; and $Z_2$ being -OH or $-NH_2$; provided that no more than three of $Xaa_3$, $Xaa_5$, $Xaa_6$, $Xaa_8$, $Xaa_{10}$, $Xaa_{11}$, $Xaa_{12}$, $Xaa_{13}$, $Xaa_{14}$, $Xaa_{15}$, $Xaa_{16}$, $Xaa_{17}$, $Xaa_{19}$, $Xaa_{20}$, $Xaa_{21}$, $Xaa_{24}$, $Xaa_{25}$, $Xaa_{26}$, $Xaa_{27}$ and $Xaa_{28}$ are Ala; and provided also that, if $Xaa_1$ is His, Arg or Tyr, then at least one of $Xaa_3$, $Xaa_4$ and $Xaa_9$ is Ala. Especially preferred compounds of formula (VII) include those described in PCT application Serial No. PCT/US98/24210, filed November 13, 1998, entitled "Novel Exendin Agonist Compounds" and having the amino acid sequences identified therein as SEQ. ID. NOS. 5-93.

According to an especially preferred aspect, provided are compounds of formula (VII) where Xaa14 is Ala, Leu, Ile, Val or pentylglycine, more preferably Leu or pentylglycine, and Xaa25 is Ala, Phe, Tyr or naphthylalanine, more preferably Phe or naphthylalanine. These compounds will be less susceptible to oxidative degration, both in vitro and in vivo, as well as during synthesis of the compound.

FORMULA VIII

Also provided are peptide compounds described in PCT Application Serial No. PCT/US98/24273, filed November 13, 1998, entitled "Novel Exendin Agonist Compounds", including compounds of the formula (VIII) [SEQ. ID. NO:30]:

$Xaa_1$ $Xaa_2$ $Xaa_3$ $Xaa_4$ $Xaa_5$ $Xaa_6$ $Xaa_7$ $Xaa_8$ $Xaa_9$ $Xaa_{10}$
$Xaa_{11}$ $Xaa_{12}$ $Xaa_{13}$ $Xaa_{14}$ $Xaa_{15}$ $Xaa_{16}$ $Xaa_{17}$ Ala $Xaa_{19}$ $Xaa_{20}$
$Xaa_{21}$ $Xaa_{22}$ $Xaa_{23}$ $Xaa_{24}$ $Xaa_{25}$ $Xaa_{26}$ $X_1$-$Z_1$;
wherein
$Xaa_1$ is His, Arg, Tyr, Ala, Norval, Val, Norleu or 4-imidazopropionyl;
$Xaa_2$ is Ser, Gly, Ala or Thr;
$Xaa_3$ is Ala, Asp or Glu;
$Xaa_4$ is Ala, Norval, Val, Norleu or Gly;
$Xaa_5$ is Ala or Thr;
$Xaa_6$ is Phe, Tyr or naphthylalanine;
$Xaa_7$ is Thr or Ser;
$Xaa_8$ is Ala, Ser or Thr;
$Xaa_9$ is Ala, Norval, Val, Norleu, Asp or Glu;
$Xaa_{10}$ is Ala, Leu, Ile, Val, pentylglycine or Met;
$Xaa_{11}$ is Ala or Ser;
$Xaa_{12}$ is Ala or Lys;
$Xaa_{13}$ is Ala or Gln;
$Xaa_{14}$ is Ala, Leu, Ile, pentylglycine, Val or Met;
$Xaa_{15}$ is Ala or Glu;
$Xaa_{16}$ is Ala or Glu;
$Xaa_{17}$ is Ala or Glu;
$Xaa_{19}$ is Ala or Val;

Xaa$_{20}$ is Ala or Arg;

Xaa$_{21}$ is Ala, Leu or Lys-NH$^\varepsilon$-R where R is Lys, Arg, C$^{1-10}$ straight chain or branched alkanoyl or cycloalleyl-alkanoyl;

Xaa$_{22}$ is Phe, Tyr or naphthylalanine;

Xaa$_{23}$ is Ile, Val, Leu, pentylglycine, tert-butylglycine or Met;

Xaa$_{24}$ is Ala, Glu or Asp;

Xaa$_{25}$ is Ala, Trp, Phe, Tyr or naphthylalanine;

Xaa$_{26}$ is Ala or Leu;

X$_1$ is Lys Asn, Asn Lys, Lys-NH$^\varepsilon$-R Asn, Asn Lys-NH$^\varepsilon$-R, Lys-NH$^\varepsilon$-R Ala, Ala Lys-NH$^\varepsilon$-R where R is Lys, Arg, C$_1$-C$_{10}$ straight chain or branched alkanoyl or cycloalkylalkanoyl

Z$_1$ is -OH,

-NH$_2$,

Gly-Z$_2$,

Gly Gly-Z$_2$,

Gly Gly Xaa$_{31}$-Z$_2$,

Gly Gly Xaa$_{31}$ Ser-Z$_2$,

Gly Gly Xaa$_{31}$ Ser Ser-Z$_2$,

Gly Gly Xaa$_{31}$ Ser Ser Gly-Z$_2$,

Gly Gly Xaa$_{31}$ Ser Ser Gly Ala-Z$_2$,

Gly Gly Xaa$_{31}$ Ser Ser Gly Ala Xaa$_{36}$-Z$_2$,

Gly Gly Xaa$_{31}$ Ser Ser Gly Ala Xaa$_{36}$ Xaa$_{37}$-Z$_2$,

Gly Gly Xaa$_{31}$ Ser Ser Gly Ala Xaa$_{36}$ Xaa$_{37}$ Xaa$_{38}$-Z$_2$ or

Gly Gly Xaa$_{31}$ Ser Ser Gly Ala Xaa$_{36}$ Xaa$_{37}$ Xaa$_{38}$ Xaa$_{39}$-Z$_2$;

Baa$_{31}$, Xaa$_{36}$, Xaa$_{37}$ and Xaa$_{38}$ are independently selected from the group consisting of Pro, homoproline, 3Hyp, 4Hyp, thioproline, N-alkylglycine, N-alkylpentylglycine and N-alkylalanine; and

Z$_2$ is -OH or -NH$_2$;

provided that no more than three of Xaa$_3$, Xaa$_4$, Xaa$_5$, Xaa$_6$, Xaa$_8$, Xaa$_9$, Xaa$_{10}$, Xaa$_{11}$, Xaa$_{12}$, Xaa$_{13}$, Xaa$_{14}$, Xaa$_{15}$, Xaa$_{16}$, Xaa$_{17}$, Xaa$_{19}$, Xaa$_{20}$, Xaa$_{21}$, Xaa$_{24}$, Xaa$_{25}$, Xaa$_{26}$, are Ala; and provided also that, if Xaa$_1$ is His, Arg, Tyr, or 4-imidazopropionyl then at least one of Xaa$_3$, Xaa$_4$ and Xaa$_9$ is Ala.

Preferred compounds of formula (VIII) include those wherein Xaa$_1$ is His, Ala, Norval or 4-imidazopropionyl. Preferably, Xaa$_1$ is His, or 4-imidazopropionyl or Ala, more preferably His or 4-imidazopropionyl.

Preferred compounds of formula (VIII) include those wherein Xaa$_2$ is Gly.

Preferred compounds of formula (VIII) include those wherein Xaa$_4$ is Ala.

Preferred compounds of formula (VIII) include those wherein Xaa$_9$ is Ala.

Preferred compounds of formula (VIII) include those wherein Xaa$_{14}$ is Leu, pentylglycine or Met.

Preferred compounds of formula (VIII) include those wherein Xaa$_{25}$ is Trp or Phe.

Preferred compounds of formula (VIII) include those wherein Xaa$_6$ is Ala, Phe or naphthylalanine; Xaa$_{22}$ is Phe or naphthylalanine; and Xaa$_{23}$ is Ile or Val.

Preferred compounds of formula (VIII) include those wherein Z$_1$ is -NH$_2$.

Preferred compounds of formula (V1I1) include those wherein Xaa$_{31}$, Xaa$_{36}$, Xaa$_{37}$ and Xaa$_{38}$ are independently selected from the group consisting of Pro, homoproline, thioproline and N-alkylalanine.

Preferred compounds of formula (VIII) include those wherein Xaa$_{39}$ is Ser or Tyr, preferably Ser.

Preferred compounds of formula (VIII) include those wherein Z$_2$ is -NH$_2$.

Preferred compounds of formula (VIII) include those 42 wherein Z$_1$ is -NH$_2$.

Preferred compounds of formula (VIII) include those wherein Xaa$_{21}$ is Lys-NH$^\varepsilon$-R where R is Lys, Arg, C$_1$-C$_{10}$ straight chain or branched alkanoyl.

Preferred compounds of formula (VIII) include those wherein X$_1$ is Lys Asn, Lys-NH$^\varepsilon$-R Asn, or Lys-NH$^\varepsilon$-R Ala where R is Lys, Arg, C$_1$-C$_{10}$ straight chain or branched alkanoyl.

Preferred compounds of formula (VIII) include those described in PCT Application Serial No. PCT/US98/24273, filed November 13, 1998, entitled "Novel Exendin Agonist Compounds" as having an amino acid sequence selected from those identified therein as SEQ. ID. NOS. 95-110.

FORMULA IX

Compounds particularly useful according to the present invention are exendin analogs with agonist activity described in U.S. Patent Application Serial No. 09/003,869, filed January 7, 1998, entitled "Use of Exendins And Agonists Thereof For The Reduction of Food Intake", including compounds of the formula (IX) [SEQ. ID. NO:31]:

Xaa$_1$ Xaa$_2$ Xaa$_3$ Gly Thr Xaa$_4$ Xaa$_5$ Xaa$_6$ Xaa$_7$ Xaa$_8$
Ser Lys Gln Xaa$_9$ Glu Glu Glu Ala Val Arg Leu
Xaa$_{10}$ Xaa$_{11}$ Xaa$_{12}$ Xaa$_{13}$ Leu Lys Asn Gly Gly Xaa$_{14}$
Ser Ser Gly Ala Xaa$_{15}$ Xaa$_{16}$ Xaa$_{17}$ Xaa$_{18}$-Z
wherein:
Xaa$_1$ is His, Arg or Tyr;
Xaa$_2$ is Ser, Gly, Ala or Thr;
Xaa$_3$ is Asp or Glu;
Xaa$_4$ is Phe, Tyr or naphthalanine;
Xaa$_5$ is Thr or Ser;
Xaa$_6$ is Ser or Thr;
Xaa$_7$ is Asp or Glu;
Xaa$_8$ is Leu, Ile, Val, pentylglycine or Met;
Xaa$_9$ is Leu, Ile, pentylglycine, Val or Met;
Xaa$_{10}$ is Phe, Tyr or naphthalanine;
Xaa$_{11}$ is Ile, Val, Leu, pentylglycine, tert-butylglycine or Met;
Xaa$_{12}$ is Glu or Asp; Xaa$_{13}$ is Trp, Phe, Tyr, or naphthylalanine;
Xaa$_{14}$, Xaa$_{15}$, Xaa$_{16}$ and Xaa$_{17}$ are independently Pro, homoproline, 3Hyp, 4Hyp, thioproline, N-alkylglycine, N-alkylpentylglycine or N-alkylalanine;
Xaa$_{18}$ is Ser, Thr or Tyr; and Z is -OH or -NH$_2$;

with the proviso that the compound does not have the formula of either SEQ. ID. NOS: 12 or 14. Preferred N-alkyl groups for N-alkylglycine, N-alkylpentylglycine and N-alkylalanine include lower alkyl groups preferably of 1 to about 6 carbon atoms, more preferably of 1 to 4 carbon atoms. Also useful in the present invention are pharmaceutically acceptable salts of the compounds of formula (IX).

Preferred exendin analogs include those wherein Xaa$_1$ is His or Tyr. More preferably Xaa$_1$ is His.

Preferred are those compounds wherein Xaa$_2$ is Gly.

Preferred are those compounds wherein Xaa$_9$ is Leu, pentylglycine or Met.

Preferred compounds include those wherein Xaa$_{13}$ is Trp or Phe.

Also preferred are compounds where Xaa$_4$ is Phe or naphthalanine; Xaa$_{11}$ is Ile or Val and Xaa$_{14}$, Xaa$_{15}$, Xaa$_{16}$ and Xaa$_{17}$ are independently selected from Pro, homoproline, thioproline or N-alkylalanine. Preferably N-alkylalanine has a N-alkyl group of 1 to about 6 carbon atoms.

According to an especially preferred aspect, Xaa$_{15}$, Xaa$_{16}$ and Xaa$_{17}$ are the same amino acid reside.

Preferred are compounds wherein Xaa$_{18}$ is Ser or Tyr, more preferably Ser.

Preferably Z is -NH$_2$.

According to one aspect, preferred are compounds of formula (VII) wherein Xaa$_1$ is His or Tyr, more preferably His; Xaa$_2$ is Gly; Xaa$_4$ is Phe or naphthalanine; Xaa$_9$ is Leu, pentylglycine or Met; Xaa$_{10}$ is Phe or naphthalanine; Xaa$_{11}$ is Ile or Val; Xaa$_{14}$, Xaa$_{15}$, Xaa$_{16}$ and Xaa$_{17}$ are independently selected from Pro, homoproline, thioproline or N-alkylalanine; and Xaa$_{18}$ is Ser or Tyr, more preferably Ser. More preferably Z is -NH$_2$.

According to an especially preferred aspect, especially preferred compounds include those of formula (IX) wherein: Xaa$_1$ is His or Arg; Xaa$_2$ is Gly; Xaa$_3$ is Asp or Glu; Xaa$_4$ is Phe or napthylalanine; Xaa$_5$ is Thr or Ser; Xaa$_6$ is Ser or Thr; Xaa$_7$ is Asp or Glu; Xaa$_8$ is Leu or pentylglycine; Xaa$_9$ is Leu or pentylglycine; Xaa$_{10}$ is Phe or naphthylalanine; Xaa$_{11}$ is Ile, Val or t-butyltylglycine; Xaa$_{12}$ is Glu or Asp; Xaa$_{13}$ is Trp or Phe; Xaa$_{14}$, Xaa$_{15}$, Xaa$_{16}$, and Xaa$_{17}$ are independently Pro, homoproline, thioproline, or N-methylalanine; Xaa$_{18}$ is Ser or Tyr: and Z is - OH or -NH$_2$; with the proviso that the compound does not have the formula of either SEQ. ID. NOS: 7 or 9. More preferably Z is -NH$_2$..

According to an especially preferred aspect, provided are compounds where Xaa$_9$ is Leu, Ile, Val or pentylglycine, more preferably Leu or pentylglycine, and Xaa$_{13}$ is Phe, Tyr or naphthylalanine, more preferably Phe or naphthylalanine. These compounds are believed to exhibit advantageous duration of action and to be less subject to oxidative degration, both in vitro and in vivo, as well as during synthesis of the compound.

FORMULA X

Also provided are compounds described in PCT Application Serial No. PCT/US98/16387, filed August 6, 1998, entitled "Novel Exendin Agonist Compounds", including compounds of the formula (X) [SEQ. ID. NO:32]:

Xaa$_1$ Xaa$_2$ Xaa$_3$ Gly Thr Xaa$_4$ Xaa$_5$ Xaa$_6$ Xaa$_7$ Xaa$_8$
Ser Lys Gln Xaa$_9$ Glu Glu Glu Ala Val Arg Leu
Xaa$_{10}$ Xaa$_{11}$ Xaa$_{12}$ Xaa$_{13}$ Leu X$_1$ Gly Gly Xaa$_{14}$

18

Ser Ser Gly Ala $Xaa_{15}$ $Xaa_{16}$ $Xaa_{17}$ $Xaa_{18}$-Z
wherein:
$Xaa_1$ is His, Arg, Tyr or 4-imidazopropionyl;
$Xaa_2$ is Ser, Gly, Ala or Thr;
$Xaa_3$ is Asp or Glu;
$Xaa_4$ is Phe, Tyr or naphthylalanine;
$Xaa_5$ is Thr or Ser;
$Xaa_6$ is Ser or Thr;
$Xaa_7$ is Asp or Glu;
$Xaa_8$ is Leu, Ile, Val, pentylglycine or Met;
$Xaa_9$ is Leu, Ile, pentylglycine, Val or Met;
$Xaa_{10}$ is Phe, Tyr or naphthylalanine;
$Xaa_{11}$ is Ile, Val, Leu, pentylglycine, tert-butylglycine or Met;
$Xaa_{12}$ is Glu or Asp;
$Xaa_{13}$ is Trp, Phe, Tyr, or naphthylalanine; $X_1$ is Lys Asn, Asn Lys, Lys-$NH^\varepsilon$-R Asn, Asn Lys-$NH^\varepsilon$-R where R is Lys, Arg, $C_1$-$C_{10}$ straight chain or branched alkanoyl or cycloalkylalkanoyl;
$Xaa_{14}$, $Xaa_{15}$, $Xaa_{16}$ and $Xaa_{17}$ are independently Pro, homoproline, 3Hyp, 4Hyp, thioproline, N-alkylglycine, N-alkylpentylglycine or N-alkylalanine;
$Xaa_{18}$ is Ser, Thr or Tyr; and Z is -OH or -$NH_2$;

with the proviso that the compound does not have the formula of either SEQ. ID. NOS. 7 or 9. Suitable compounds of formula (X) include compounds described in PCT Application Serial No. PCT/US98/16387, filed August 6, 1998, entitled "Novel Exendin Agonist Compounds" having the amino acid sequences of SEQ. ID. NOS. 37-40 therein.

Preferred exendin analogs of formula (X) include those wherein $Xaa_1$ is His, Tyr or 4-imidazopropionyl. More preferably, $Xaa_1$ is His or 4-imidazopropionyl.

Preferred are those compounds of formula (X) wherein $Xaa_2$ is Gly.

Preferred are those compounds of formula (X) wherein $Xaa_9$ is Leu, pentylglycine or Met.

Preferred are those compounds of formula (X) wherein $Xaa_{13}$ is Trp or Phe.

Preferred are those compounds of formula (X) wherein

$X_1$ is Lys Asn, or Lys-$NH^\varepsilon$-R Asn, where R is Lys, Arg, $C_1$-$C_{10}$ straight chain or branched alkanoyl.

Also preferred are compounds of formula (X) wherein $Xaa_4$ is Phe or naphthylalanine; $Xaa_{10}$ is Phe or naphthylalanine; $Xaa_{11}$ is Ile or Val and $Xaa_{14}$, $Xaa_{15}$, $Xaa_{16}$ and $Xaa_{17}$ are independently selected from Pro, homoproline, thioproline or N-alkylalanine According to an especially preferred aspect, $Xaa_{18}$ is Ser or Tyr. Preferred are those such compounds wherein $Xaa_{18}$ is Ser. Preferably, Z is -$NH_2$.

According to one preferred aspect, preferred are compounds of formula (X) wherein $Xaa_4$ is Phe or naphthylalanine; $Xaa_{10}$ is Phe or naphthylalanine; $Xaa_{11}$ is Ile or Val, $X_1$ is Lys Asn, or Lys-$NH^\varepsilon$-R Asn, where R is Lys, Arg, $C_1$-$C_{10}$ straight chain or branched alkanoyl and $Xaa_{14}$, $Xaa_{15}$, $Xaa_{16}$ and $Xaa_{17}$ am independently selected from Pro, homoproline, thioproline or N-alkylalanine.

Exendins and exendin agonists that are peptides, such as exendin analogs, described herein may be prepared through peptide purification as described in, for example, Eng, et al., J. Biol. Chem. 265:20259-62, 1990; and Eng, et al., J. Biol. Chem. 267:7402-05, 1992. Alternatively, exendins, GLP-1s, and agonist analogs that are peptides may be prepared by methods known to those skilled in the art, for example, as described in Raufman, et al., J. Biol. Chem. 267: 21432-37, 1992), using standard solid-phase peptide synthesis techniques and preferably an automated or semiautomated peptide synthesizer as previously described and is well known in the art.

Peptide molecules of the invention may also be prepared using recombinant DNA techniques, using methods now known in the art. See, *e.g.*, Sambrook et al., Molecular Cloning: A Laboratory Manual, 2d Ed., Cold Spring Harbor (1989), with any necessary chemical modifications made to the molecules in additional steps as known in the art. Alternatively, such compounds may be prepared by homogeneous phase peptide synthesis methods. Non-peptide compounds useful in the present invention may be prepared by art-known methods. For example, phosphate-containing amino acids and peptides containing such amino acids, may be prepared using methods known in the art. See, *e.g.*, Bartlett and Landen, Biorg. Chem. 14:356-377 (1986). Methods for making and/or purifying GLP-1 and its agonist analogs, as discussed previously, can also be utilized to make and/or purify exendins and their agonist analogs.

Also included in the present invention are peptide sequences having greater than 70% or , and preferably greater than 80, 90, or 95% amino acid sequence identity to SEQ ID NOs: 1, and 14, as well as truncated sequences thereof. As used herein, sequence identity refers to a comparison made between two molecules using standard algorithms well known in the art. The preferred algorithm for calculating sequence identity for the present invention is the Smith-Waterman algorithm, for example, SEQ ID NO: 1 [*i.e.,* GLP-1(1-37)], SEQ ID NO: 14 [exendin- 4 ] can be used as the reference sequences to define the percentage identity of homology over their length. The choice of parameter values for matches, mismatches, and insertions or deletions is arbitrary, although some parameter values have been found to yield more biologically realistic results than others. One preferred set of parameter values for the Smith-Waterman algorithm is set forth in the "maximum similarity segments" approach, which uses values of 1 for a matched residue and -⅓ for a mismatched residue (a residue being either a single nucleotide or single amino acid). Waterman, Bull. Math. Biol. 46; 473 (1984). Insertions and deletions (indels), x, are weighted as $x_k = 1 + ⅓k$, where k is the number of residues in a given insert or deletion. *Id.*

For instance, a sequence that is identical to the 37-amino acid residue sequence of SEQ ID NO: 1, except for 18 amino acid substitutions and an insertion of 3 amino acids, would have a percent identity given by:

$$[(1 \times 37 \text{ matches}) - (^1/_3 \times 18 \text{ mismatches})$$

$$- (1 + 3/3 \text{ indels})] / 37 = 78\% \text{ "identity."}$$

This algorithm can be used with any amino acid sequence to determine sequence homology. For purposes of determining homology, truncation of the mature sequence should be disregarded. Sequences having lesser degrees of homology, comparable bioactivity, and equivalent expression characteristics are considered equivalents.

The biological activity of a GLP-1 agonist and/or analog can be determined by *in vitro* and *in vivo* animal models and human studies, as is well known to the skilled artisan. GLP-1 biological activity can be determined by standard methods, in general, by receptor binding activity screening procedures, which involve providing appropriate cells that express the GLP-1 receptor on their surface, for example, insulinoma cell lines such as RINmSF cells or INS-1 cells. *See* Mojsov, Int. J. Peptide Protein Res. 40; 333 (1992) and EP 0708179 A2. GLP-1 receptors are cell-surface proteins found, for example, on insulin-producing pancreatic β-cells; the GLP-1 (7-36) receptor has been characterised in the art. Additional receptors at which GLP-1 and exendins act are also thought to exist, and may mediate effects by which the instant invention is operative. Methods of determining whether a chemical or peptide binds to or activates a particular GLP-1 receptor are known to the skilled artisan. For example, U.S. Patent Nos. 6,051,689, 5,846,747, and 5,670,360 describe GLP-1 receptors, as well as methods for using them. Cells that are engineered to express a GLP-1 receptor also can be used. In addition to measuring specific binding of tracer to membrane using radioimmunoassay methods, cAMP activity or glucose dependent insulin production can also be measured. In one method, a polynucleotide encoding a GLP-1 receptor is employed to transfect cells so that they express the GLP-1 receptor protein. Thus, for example, these methods may be employed for screening for a receptor agonist by contacting such cells with compounds to be screened and determining whether such compounds generate a signal (*i.e.*, activate the receptor). Other screening techniques include the use of cells that express the GLP-1 receptor, for example, transfected CHO cells, in a system to measure extracellular pH or ionic changes caused by receptor activation. For example, potential agonists may be contacted with a cell that expresses the GLP-1 protein receptor and a second messenger response (e.g., signal transduction or ionic or pH changes), may be measured to determine whether the potential agonist is effective.

The molecules of the present invention may be used in combination with a suitable pharmaceutical carrier. Such compositions comprise a therapeutically effective amount of the polypeptide, and a pharmaceutically acceptable carrier or excipient The compounds of this invention can be administered in any effectively pharmaceutically acceptable form to animals, including human subjects, e.g. in topical, lavage, oral, suppository, parenteral, injectible and/or infusible dosage forms, as a topical, buccal, sublingual, pulmonary or nasal spray, or in any other manner effective to deliver the agents. The route of administration will preferably be designed to optimize delivery and/or localization of the agents, and for peptide molecules of the invention, is preferably via a subcutaneous or other parenteral injection route, or transmucosal delivery.

In addition to administration with conventional carriers, active ingredients may be administered by a variety of specialized delivery drug techniques which are known to those of skill in the art, such as portable infusion pumps.

Suitable formulations for the peptide molecules of the invention are disclosed in U.S. Serial No. 09/899,330 and related applications. Additional formulations for administration may be made in accordance with methods and amounts known in the art, such as set forth in Remington's Pharmaceutical Sciences. 18th Ed., Wiley Publishing (1990).

The peptides of the present invention are administered along with a pharmaceutically acceptable carrier in an amount sufficient to prevent or treat nephropathy. The compounds of this invention have extremely low toxicity and a low degree of side effects even at high doses. The dosing range of the compounds of this invention will vary depending on a number of factors, such as route and manner of administration, i.e. sustained release or continuous, such as intravenous infusion or subcutaneous infusion, desired dosing schedule, etc.

Although not limited to the following ranges and provided only as an illustration, exemplary dose ranges for peptides of the invention can include 0,001 pmol/kg to 500 nmol/kg per day depending on the composition selected. A lower limit of a dosage range can be about 0.001 pmol/kg, 0.01 pmol/kg, 0.1 pmol/kg, 1 pmol/kg, 10 pmol/kg, or 100 pmol/kg. An upper dosage range can be about 10 pmol/kg, 100 pmol/kg, 1 nmol/kg, 10 nmol/kg, 100 nmol/kg, 250 nmol/kg or 500 nmol/kg. The desired dose will vary depending on the selected active composition and its relative potency compared to e.g., GLP-1 and exendin. The desired dose will also depend upon other factors including bioavailability, the route of administration and the formulation. For example, continuous infusion as well as bolus doses and sustained release formulations are contemplated and may include administration of the peptide in liquid, gel, semi-solid or solid form.

Alternatively, doses from about 0.0005 μg/kg/dose to about 12000 μg/kg/dose, depending on mode of administration, can be used to achieve therapeutic plasma levels (at least 5 pg/ml, preferably at least 40 pg/ml). For molecules having potency similar to exendin-4, preferably peak plasma levels will not exceed about 500pg/ml, more preferably about 250 pg/ml, and most preferably about 150 pg/ml.

Administered parenterally, exendins and agonists in an amount from about 0.001 μg/kg/dose to about 1.0 μg/kg/dose produce therapeutic effects. In one embodiment, the composition is administered peripherally.

Exemplary doses for continuous infusion by intravenous (I.V.) can be about 0.1 pmol/kg/min to 10 pmol/kg/min and by subcutaneous (s.c.) about 0.1 pmol/kg/min to 75 pmol/kg/min., and for single injection (bolus) by I.V. about 0.1 nmol/kg to 2.0 nmol/kg and s.c. about 0.1 nmol/kg to 100 nmol/kg. The foregoing doses may be administered as a single dose per day or may be divided into multiple doses for administration per day. The peptides of this invention may be administered once to several times daily.

While a preferred method of administration of a GLP-1 peptide may be through a continuous application, other forms of delivery as described above are also contemplated. However, an exemplary dosing rate can be within a range of from about 1 to about 10 pmol/kg per minute of GLP-1 delivered by sustained release subcutaneous, intramuscular, interperitoneal, injected depot with sustained release, deep lung insufflation, as well as by intravenous, buccal, patch or other sustained release delivery methods. Degradation-resistant GLP-1 analogs, derivatives or variants, exendins, analogs, derivatives or variants, and other molecules of the invention need not be delivered continuously, but are suitable for bolus or sustained release dosing and may be at doses much lower than those described.

Other drugs besides compositions of the invention which are compatible with the carrier ingredients may also be incorporated into the pharmaceutical formulations. Such drugs may be readily ascertained by those of ordinary skill in the art and may include, for instance, antiinflammatory agents, diuretics, vasodilators, etc.

It is understood that the present invention contemplates the use of not only the above-stated active forms of the compositions of the invention, but also includes the prodrugs (proforms) which metabolize to the compound and biologically active salt forms thereof, as well as optical isomers which provide the same pharmaceutical results.

The compositions of the invention may also be used in combination with agents known in the art that enhance the half-life *in vivo* of peptide in order to enhance or prolong the biological activity of the peptide. For example, a molecule or chemical moiety may be covalently linked to the composition of the present invention before administration thereof. Alternatively, the enhancing agent may be administered concurrently with the composition. Still further, the agent may comprise a molecule that is known to inhibit the enzymatic degradation of the compositions of the invention that may be administered concurrently with or after administration of the composition. Such a molecule may be administered, for example, orally, by injection, or any other means known in the art.

While there is no hard and fast rule as to when or how often GLP-1 must be administered in accordance with this invention to prevent nephropathy, as a general guideline GLP-1 may be administered to a patient that has two or more risk factors present for developing the disease, including but not limited to insulin resistance, diabetes, history of uncontrolled high blood pressure, kidney disease, increased creatinine clearance level, proteinuria, and non-Caucasian racial decent.

The following examples are provided as illustrations of the utility of the peptide molecules of the invention, and are not intended to be limiting.

## EXAMPLES

Dahl S rats are insulin-resistant and rapidly develop severe hypertension and renal injury when fed a high salt diet. The increase in mean arterial pressure (MAP) is associated with sodium retention that can be prevented by servocontrolling total body fluid volume or by using diuretics. Dahl S rats exhibit many phenotypic traits associated with salt-sensitive hypertension in man. Specifically, they are salt-sensitive, insulin-resistant and hyperlipidemic. They also develop glomerulosclerosis following the development of hypertension. The type of renal injury seen in Dahl S rats fed a high salt diet resembles that seen in patients with diabetic nephropathy, and in hypertensive African-Americans, in whom the incidence of end-stage renal disease is 16 times higher than that seen in Caucasian hypertensive patients.

Example 1: Methods

Experiments were performed on male Dahl sensitive/Jr (Dahl S) rats maintained on a low salt diet (0.1 % NaCl) from birth to prevent the development of hypertension. When the rats were 9 weeks old, they were anesthetized with an i.m. injection of ketamine (40 mg/kg), xylazine (2.5 mg/kg), and acepromazine (0.6 mg/kg) and catheters were implanted in the femoral artery and vein for chronic measurement of MAP (mean arterial pressure) and i.v. infusion (10 ml/day). The rats received an i.m. injection of enrofloxacin (Baytril, 2.5 mg/kg) to prevent infections and were given 4-5 days to recover from surgery.

Evaluation of Effects of rGLP-1 on MAP and Renal Dysfunction

MAP was measured on 3 consecutive days and a blood sample and an overnight urine sample was collected during the control period while the rats were maintained on a low salt diet (0.4 NaCl) and infused with the vehicle for recombinant glucagon-like peptide-1(7-36)amide (rGLP-1) (5% mannitol solution on 0.9% saline) at a rate of 10 ml/day. The rats were then switched to a high salt diet (8% NaCl) for 14 days. One group of rats received a continuous i.v. infusion of rGLP-1 at a dose of 1 ug/kg/min, while the other group of rats was infused with vehicle. MAP was directly recorded from the catheter implanted in the femoral artery on days 3, 7, 10 and 14 of the high salt diet using a computerized data acquisition system (WINDAQ software, DataQ Instruments Inc. Akron, OH) at a sample rate of 300 Hz between 11:00 AM and 3:00 PM while the rats were conscious and freely moving in their home cages. MAP was averaged over 1-min periods and converted to a mean value for the recording session. In addition, a blood sample was collected from the arterial catheter for measurement of the plasma creatinine concentration and an overnight urine sample was collected for measurement of proteinuria and microalbuminuria on days 7 and 14 after starting the high salt diet. Urine protein concentration was determined by the Bradford method (Bio-Rad Laboratories Hercules, CA) with bovine serum albumin as the standard. Urine albumin concentration was measured using the albumin blue 580 method (Molecular Probes, Eugene, OR).

Histological Evaluation of the Kidney

At the end of the experiment, the rats were anesthetized with pentobarbital (60 mg/Kg, i.p.), and the kidney was collected, weighed and fixed in a 5% buffered formalin solution. The tissues were later embedded in paraffin, sectioned and stained with Mason's trichrome stain and examined by light microscopy. The degree of glomerulosclerosis was scored as previously described by Raij et al on a scale of 0-4 based on the percentage of glomerular capillary area filled with mesangial matrix. A score of 0 indicates no damage, a score of 2 indicates that 50% of the glomerular capillaries area is filled with matrix, and a score of 4 indicates complete closure of all capillaries within a given glomerulus. The kidney sections were also examined for the degree of renal interstitial fibrosis and the percentage of medullary area occupied by protein casts was determined using a Metamorph imaging program on at least 10 regions per kidney section.

Statistical Analysis

Mean values $\pm$ SE are presented. The significance of differences in mean values measured in the vehicle- and rGLP-1 treated groups were analyzed using a two-way ANOVA for repeated measurements followed by the Duncan's multiple-range test or an unpaired t-test. The significance of differences within the group was tested using an ANOVA for repeated measures. A P value <0.05 was considered statistically significant.

Example 2: Effect of rGLP-1 on the Development of Hypertension

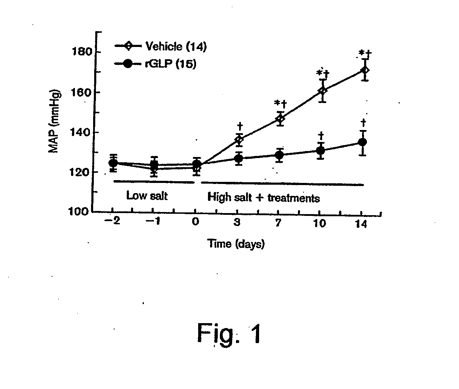

Rats were maintained on a low salt diet (0.4 % NaCl) during the 3 day control period. The rats were then switched to a high salt diet (8% NaCl) and received either rGLP-1 (1 $\mu$g/kg/min) or vehicle. Figure 1 indicates the development of hypertension in vehicle-treated rats upon initiation of a high salt diet, and the protection afforded by administration of GLP-1. Numbers in parentheses indicate the number of rats studied. * indicates P<0.05 versus vehicle treatment and + indicates P<0.05 versus control value measured on a low salt diet. Baseline MAP measured while the rats were fed a low salt diet was similar in the rats subsequently treated with rGLP-1 or vehicle and averaged 122 $\pm$ 2 mmHg. MAP increased to 174 $\pm$ 6 mmHg in the vehicle-treated Dahl S rats fed a high salt diet for 14 days. In contrast, the rise in MAP was significantly attenuated and MAP only rose to 136 $\pm$ 7 mmHg in the rats infused with rGLP-1.

Example 3: Effect of rGLP-1 on Renal Dysfunction

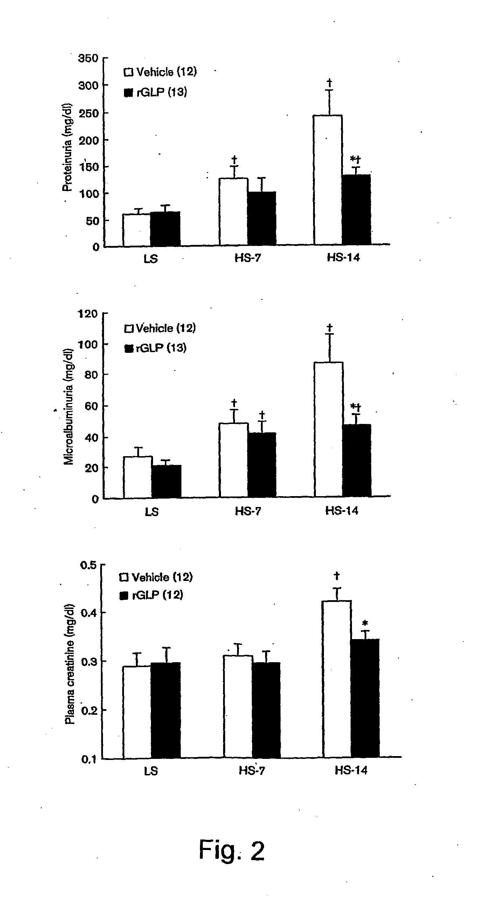

The effects of rGLP-1 on the development of proteinuria, microalbuminuria and plasma creatinine concentration (indicators of renal damage and nephropathy) in Dahl S rats fed a high salt diet are presented in Figure 2. Rats were maintained on a low salt diet (0.4 % NaCl) during the 3 day control period. The rats were then switched to a high salt diet (8 % NaCl) and received either rGLP-1 (1 $\mu$g/kg/min) or vehicle. LS: low salt diet, HS-7 or -14: 7 or 14 days after high salt diet. Numbers in parentheses indicate the number of rats studied. * indicates P<0.05 versus vehicle treatment and + indicates P<0.05 versus control value measured on a low salt diet. The excretion of protein and albumin increased significantly after 14 days on a high salt diet in Dahl S rats treated with vehicle (Figs.2A and 2B). This was associated with a significant increase in plasma creatinine concentration, an index of glomerular filtration rate (GFR, Figure 2C). Chronic administration of rGLP-1 significantly attenuated the increase in urinary excretion of protein and albumin by 62% and 68%, respectively (Figs. 2A and 2B). rGLP-1 also reduced the rise in plasma creatinine concentration in Dahl S rats fed a high salt diet by 62% (Figure 2C).

Example 4: Reduction in Renal End-Organ Damage

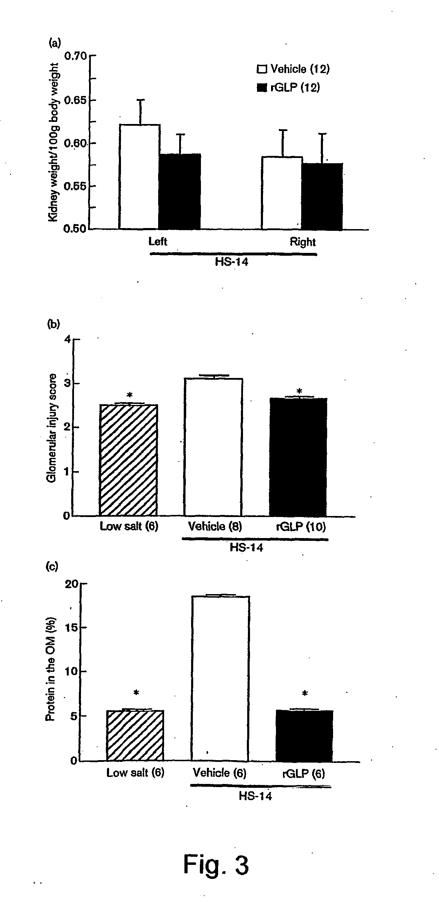

The effects of rGLP-1 on hypertension-induced renal end organ damage in Dahl S rats fed a high salt diet for 14 days (HS-14) is presented in Figure 3. Rats were treated with rGLP-1 or vehicle. Low salt (LS), in Figures 3B and 3C, indicates results obtained from a separate group of normotensive Dahl S rats maintained on a low salt (0.4 % NaCl) diet throughout the study. Numbers in parentheses indicate the number of rats studied. * indicates P<0.05 versus control Dahl S rats fed a high salt diet and treated with vehicle. Chronic treatment of Dahl S rats with rGLP-1 reduced the kidney weight (Fig. 3A), an index of renal hypertrophy. In vehicle-treated Dahl S rats there was marked expansion of the mesangial matrix in nearly every glomerulus examined and the overall glomerular injury score averaged 3.1, indicating that more than 75% of the area of glomerular capillaries was filled with matrix. Chronic treatment of the rats with rGLP-1 significantly reduced the degree of matrix expansion and the glomerular injury (Fig. 3B). For comparison, we also examined the degree of glomerular injury in a group of normotensive Dahl S rats maintained on a low salt diet for 14 days. The glomerular injury score was not significantly different from that seen in Dahl S rats that were treated with rGLP-1 and fed the high salt diet ($2.5 \pm 0.04$ vs. $2.64 \pm 0.05$, Fig. 3B). These results are consistent with previous reports that Dahl S rats exhibit a high degree of glomerular damage even when maintained on a low salt diet to minimize the development of hypertension [26]. Similarly, there was marked necrosis of renal tubules and formation of protein casts in the outer medulla of vehicle-treated rats (Fig. 3C). In contrast, the number of protein casts and the degree of tubular necrosis was greatly reduced in the outer medulla of Dahl S rats infused with rGLP-1 (Fig. 3C).

Example 4: Improvement in Endothelial Function

Thoracic aorta of vehicle- and GLP-1-treated rats were collected and placed in cold physiological saline solution (PSS) containing (in mmol/l): 119 NaCL, 4.7 KCl, 1.17 $MgSO_4$, 1.6 $CaCl_2$. 12 $NaHCO_3$, 1.18 $NaH_2PO_4$, 0.03 EDTA, 10 glucose and 10 HEPES (pH 7:4). The connective tissue and two rings (about 5 mm in length) were prepared from the aorta of each rat. The rings were mounted in an organ bath on tungsten wires connected to force transducers (Model FT03E, Grass Instruments, Rhode Island). The vessels were bathed in PSS, bubbled with 95% $O_2$ and 5% $CO_2$ and maintained at 37°C. Data were acquired using a computerized data acquisition system (WINDAQ software). The rings were preloaded with 2-3g tension and were allowed to equilibrate for 60-90 min until a reproducible contraction was achieved following addition of a depolarizing concentration of 60mmol/l KCl to the bath. Vessels were preconstriced with norepinephrine (NE, $10^{-7}$ mol/l). Then, cumulative dose-response curves to acetylcholine (Ach, from $10^{-9}$ to $10^{-4}$ mol/l) or a NO donor, DEA NON-Oate (from $10^{-9}$ to $10^{-4}$ mol/l), were constructed. Between each dose-response study, rings were bathed in fresh PSS and re-equilibrated for 60 min. Control experiments were performed on aortic rings from a group of normotensive Sprague-Dawley (SD) rats.

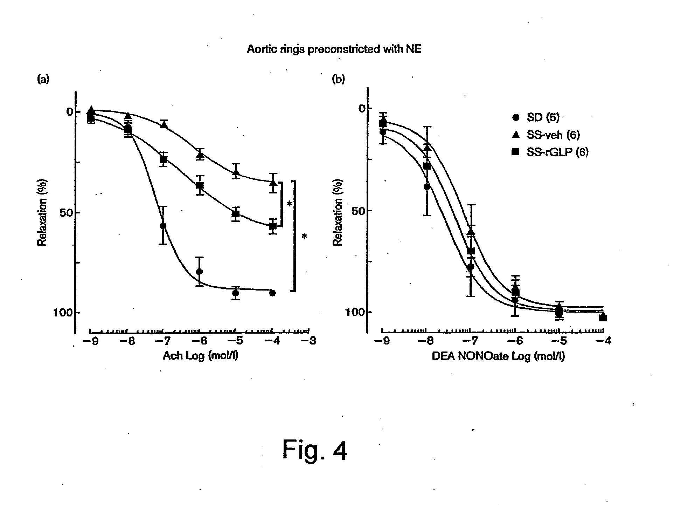

Figure 4 indicates the effects of GLP-1 to help restore endothelial function. In normotensive SD rats, Ach reduced the tension of aortic ringes preconstricted with NE by 90%. The vasodilator response to ACh was markedly less in aortic rings prepared from vehicle-treated Dahl S rats fed a high salt diet. Chronic treatment of the Dahl S rats with GLP-1 partially restored the endothelial function. The vasodilator response to Ach in the GLP-1 treated rats was nearly twice that seen in the vehicle-treated raths ($57 \pm 4$ vs. $35 \pm 5$% relaxation at $10^{-4}$M, Fig. 4a). The vasodilator responses to the NO donor were similar across aortic rings from all rats (Fig. 4b).
These examples demonstrate that an exemplary molecule of the invention, GLP-1, has antihypertensive and renoprotective effects.

**Claims**

1. Use of a GLP-1, an exendin, or an agonist analog having at least 70% amino acid sequence identity with GLP-1 (7-36) or exendin-4, in the manufacture of a medicament for the prevention or treatment of nephropathy.

2. Use of a GLP-1, an exendin, or an agonist analog having at least 70% amino acid sequence identity with GLP-1 (7-36) or exendin-4, in the manufacture of a medicament for preventing progression of nephropathy to end stage renal disease (ESRD) in a subject having nephropathy.

3. Use of a GLP-1, an exendin, or an agonist analog having at least 70% amino acid sequence identity with GLP-1 (7-36) or exendin-4, in the manufacture of a medicament for reducing proteinuria in a subject in need thereof.

4. Use of a GLP-1, an exendin, or an agonist analog having at least 70% amino acid sequence identity with GLP-1 (7-36) or exendin-4, in the manufacture of a medicament for preventing or slowing progression of glomerulosclerosis in a subject in need thereof.

5. The use according to any of claims 1 through 4 wherein the subject also suffers from insulin resistance, diabetes, or hypertension.

6. The use according to any of claims 1 through 5 wherein the GLP-1 is GLP-1 (7-36) $NH_2$.

7. The use according to any of claims 1 through 5 wherein the exendin is exendin-3 or exendin-4.

8. The use according to any of clams 1 through 7 wherein the composition is administered in a dose of from about 0.001 pmol/kg to 20 nmol/kg.

9. The use according to any of claims 1 through 8 wherein the composition is administered in a dose of from about 0.001 ug/kg/dose to about 1.0 ug/kg/dose.

10. The use according to any of claims 1 through 9 wherein the composition is administered in a dose sufficient to achieve a therapeutic plasma level of at least 40 pg/ml.

11. The use according to any of claims 1 through 10 wherein:

   a) the composition is administered peripherally;
   b) the composition is administered intravenously in a dose of from about 0.1 pmol/kg/min up to about 10 pmol/kg/min; or
   c) the composition is administered subcutaneously in a dose of from about 0.1 pmol/kg/min to 75 pmol/kg/min.

12. The use according to any one of claims 1 to 11 wherein the agonist analog has at least 80% amino acid sequence identity with GLP-1 (7-36) or exendin-4.

13. The use according to any one of claims 1 to 12, wherein the agonist analog has at least 90% amino acid sequence identity with GLP-1 (7-36) or exendin-4.

14. The use according to any one of claims 1 to 13, wherein the agonist analog has at least 95% amino acid sequence identity with GLP-1 (7-36) or exendin-4.

15. A GLP-1, an exendin, or an agonist analog having at least 70% amino acid sequence identity with GLP-1 (7-36) or exendin 4, for use in:

   a) the prevention or treatment of nephropathy;
   b) preventing progression of nephropathy to ESRD in a subject having nephropathy;
   c) reducing proteinuria in a subject in need thereof; or
   d) preventing or slowing progression of glomerulosclerosis in a subject in need thereof.

**Patentansprüche**

1. Verwendung eines GLP-1, eines Exendins oder eines Agonisten-Analogons, das mindestens 70% Aminosäuresequenzidentität mit GLP-1 (7-36) oder Exendin-4 hat, zur Herstellung eines Medikaments zur Prävention oder Behandlung von Nephropathie.

2. Verwendung eines GLP-1, eines Exendins oder eines Agonisten-Analogons, das mindestens 70% Aminosäuresequenzidentität mit GLP-1 (7-36) oder Exendin-4 hat, zur Herstellung eines Medikaments zur Prävention des Fortschreitens von Nephropathie zur Nierenerkrankung im Endstadium (ESRD) bei einem Subjekt, das Nephropathie hat.

3. Verwendung eines GLP-1, eines Exendins oder eines Agonisten-Analogons, das mindestens 70% Aminosäuresequenzidentität mit GLP-1 (7-36) oder Exendin-4 hat, zur Herstellung eines Medikaments zur Verringerung von Proteinurie bei einem Subjekt, das dies benötigt.

4. Verwendung eines GLP-1, eines Exendins oder eines Agonisten-Analogons, das mindestens 70% Aminosäuresequenzidentität mit GLP-1 (7-36) oder Exendin-4 hat, zur Herstellung eines Medikaments zur Prävention oder Verlangsamung des Fortschreitens von Glomerulosklerose bei einem Subjekt, das dies benötigt.

5. Verwendung gemäß einem der Ansprüche 1 bis 4, wobei das Subjekt auch an Insulin-Resistenz, Diabetes oder Bluthochdruck leidet.

6. Verwendung gemäß einem der Ansprüche 1 bis 5, wobei das GLP-1 GLP-1 (7-36) $NH_2$ ist.

7. Verwendung gemäß einem der Ansprüche 1 bis 5, wobei das Exendin Exendin-3 oder Exendin-4 ist.

8. Verwendung gemäß einem der Ansprüche 1 bis 7, wobei die Zusammensetzung in einer Dosis von etwa 0,001 pmol/kg bis 20 nmol/kg verabreicht wird.

9. Verwendung gemäß einem der Ansprüche 1 bis 8, wobei die Zusammensetzung in einer Dosis von etwa 0,001 $\mu$g/kg/Dosis bis etwa 1,0 $\mu$g/kg/Dosis verabreicht wird.

10. Verwendung gemäß einem der Ansprüche 1 bis 9, wobei die Zusammensetzung in einer Dosis verabreicht wird, die ausreicht, um ein therapeutisches Plasmalevel von mindestens 40 pg/ml zu erreichen.

11. Verwendung gemäß einem der Ansprüche 1 bis 10, wobei:

a) die Zusammensetzung peripher verabreicht wird;
b) die Zusammensetzung intravenös in einer Dosis von etwa 0,1 pmol/kg/min bis zu etwa 10 pmol/kg/min verabreicht wird; oder
c) die Zusammensetzung subkutan in einer Dosis von etwa 0,1 pmol/kg/min bis 75 pmol/kg/min verabreicht wird.

12. Verwendung gemäß einem der Ansprüche 1 bis 11, wobei das Agonisten-Analogon mindestens 80% Aminosäuresequenzidentität mit GLP-1 (7-36) oder Exendin-4 hat.

13. Verwendung gemäß einem der Ansprüche 1 bis 12, wobei das Agonisten-Analogon mindestens 90% Aminosäuresequenzidentität mit GLP-1 (7-36) oder Exendin-4 hat.

14. Verwendung gemäß einem der Ansprüche 1 bis 13, wobei das Agonisten-Analogon mindestens 95% Aminosäuresequenzidentität mit GLP-1 (7-36) oder Exendin-4 hat.

15. GLP-1, ein Exendin oder ein Agonisten-Analogon, das mindestens 70% Aminosäuresequenzidentität mit GLP-1 (7-36) oder Exendin 4 hat, zur Verwendung bei:

a) der Prävention oder Behandlung von Nephropathie;
b) der Prävention des Fortschreitens von Nephropathie zu ESRD bei einem Subjekt, das Nephropathie hat;
c) zum Verringern von Proteinurie bei einem Subjekt, das dies benötigt; oder
d) zur Prävention oder zum Verlangsamen des Fortschreitens von Glomerulosklerose bei einem Subjekt, das dies benötigt.

**Revendications**

1. Utilisation d'un peptide GLP-1, d'une exendine, ou d'un analogue agoniste présentant une séquence d'acides aminés identique à un degré d'au moins 70 % à celle du GLP-1(7-36) ou de l'exendine-4, dans la fabrication d'un médicament destiné à la prévention ou au traitement d'une néphropathie.

**2.** Utilisation d'un peptide GLP-1, d'une exendine, ou d'un analogue agoniste présentant une séquence d'acides aminés identique à un degré d'au moins 70 % à celle du GLP-1(7-36) ou de l'exendine-4, dans la fabrication d'un médicament destiné à prévenir, chez un sujet atteint de néphropathie, la progression de la néphropathie vers l'insuffisance rénale terminale (ESRD).

**3.** Utilisation d'un peptide GLP-1, d'une exendine, ou d'un analogue agoniste présentant une séquence d'acides aminés identique à un degré d'au moins 70 % à celle du GLP-1(7-36) ou de l'exendine-4, dans la fabrication d'un médicament destiné à faire baisser le taux de protéinurie chez un sujet qui en a besoin.

**4.** Utilisation d'un peptide GLP-1, d'une exendine, ou d'un analogue agoniste présentant une séquence d'acides aminés identique à un degré d'au moins 70 % à celle du GLP-1(7-36) ou de l'exendine-4, dans la fabrication d'un médicament destiné à prévenir ou ralentir la progression d'une glomérulosclérose chez un sujet qui en a besoin.

**5.** Utilisation conforme à l'une des revendications 1 à 4, pour laquelle le sujet souffre également de résistance à l'insuline, de diabète ou d'hypertension.

**6.** Utilisation conforme à l'une des revendications 1 à 5, pour laquelle le peptide GLP-1 est le GLP-1(7-36)NH$_2$.

**7.** Utilisation conforme à l'une des revendications 1 à 5, pour laquelle l'exendine est l'exendine-3 ou l'exendine-4.

**8.** Utilisation conforme à l'une des revendications 1 à 7, pour laquelle la composition sera administrée en une dose d'environ 0,001 pmol/kg à 20 nmol/kg.

**9.** Utilisation conforme à l'une des revendications 1 à 8, pour laquelle la composition sera administrée en une dose d'environ 0,001 μg/kg/dose à environ 1,0 μg/kg/dose.

**10.** Utilisation conforme à l'une des revendications 1 à 9, pour laquelle la composition sera administrée en une dose suffisante pour parvenir à un taux plasmatique thérapeutique d'au moins 40 pg/mL.

**11.** Utilisation conforme à l'une des revendications 1 à 10, pour laquelle :

a) la composition sera administrée par voie périphérique ;
b) la composition sera administrée en intraveineuse, en une dose d'environ 0,1 pmol/kg/min à environ 10 pmol/kg/min ;
c) ou la composition sera administrée en sous-cutanée, en une dose d'environ 0,1 pmol/kg/min à environ 75 pmol/kg/min.

**12.** Utilisation conforme à l'une des revendications 1 à 11, pour laquelle l'analogue agoniste présente une séquence d'acides aminés qui est identique à un degré d'au moins 80 % à celle du GLP-1(7-36) ou de l'exendine-4.

**13.** Utilisation conforme à l'une des revendications 1 à 12, pour laquelle l'analogue agoniste présente une séquence d'acides aminés qui est identique à un degré d'au moins 90 % à celle du GLP-1(7-36) ou de l'exendine-4.

**14.** Utilisation conforme à l'une des revendications 1 à 13, pour laquelle l'analogue agoniste présente une séquence d'acides aminés qui est identique à un degré d'au moins 95 % à celle du GLP-1(7-36) ou de l'exendine-4.

**15.** Peptide GLP-1, exendine, ou analogue agoniste présentant une séquence d'acides aminés identique à un degré d'au moins 70 % à celle du GLP-1(7-36) ou de l'exendine-4, pour utilisation dans le but :

a) de prévenir ou traiter une néphropathie ;
b) de prévenir, chez un sujet atteint de néphropathie, la progression de la néphropathie vers l'insuffisance rénale terminale ;
c) de faire baisser le taux de protéinurie chez un sujet qui en a besoin ;
d) ou de prévenir ou ralentir la progression d'une glomérulosclérose chez un sujet qui en a besoin.

Fig. 1

Fig. 2

Fig. 3

Aortic rings preconstricted with NE

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5512549 A, Chen
- US 5574008 A, Johnson
- US 5545618 A, Buckley
- US 5118666 A
- US 5120712 A
- WO 0198331 A
- WO 0248192 A
- US 276772 A
- US 5981488 A
- US 5705483 A
- WO 9111457 A
- US 6528486 B
- EP 0709179 A2
- US 9816387 W
- US 05540497 P
- US 9824210 W
- US 06544297 P
- US 9824273 W
- US 06602997 P
- US 9714199 W
- US 69495496 A
- US 9800449 W
- US 03490597 P
- US 09554533 B
- US 554531 A
- US 00386998 A
- EP 0708179 A2
- US 6051689 A
- US 5846747 A
- US 5670360 A
- US 09899330 B

**Non-patent literature cited in the description**

- **ORSKOV et al.** *Diabetes,* 1993, vol. 42, 658-61
- **D'ALESSIO et al.** *J. Clin. Invest.,* 1996, vol. 97, 133-38
- **WILLIAMS B et al.** *J Clin Endocrinol Metab,* 1996, vol. 81 (1), 327-32
- **WETTERGREN A et al.** *Dig Dis Sci,* 1993, vol. 38 (4), 665-73
- **SCHJOLDAGER BT et al.** *Dig Dis Sci,* 1989, vol. 34 (5), 703-8
- **O'HALLORAN DJ et al.** *J Endocrinol,* 1990, vol. 126 (1), 169-73
- **MOJSOV, S.** *Int. J. Peptide Protein Research,* 1992, vol. 40, 333-343
- **HJORTH et al.** *J. Biol. Chem.,* 1994, vol. 269, 30121
- **SIEGEL et al.** *Amer. Diabetes Assoc, 57th Scientific Session,* 1997
- **HARETER et al.** *Amer. Diabetes Assoc. 57th Scientific Session,* 1997
- **ADELHORST et al.** *J. Biol. Client.,* 1994, vol. 269, 6275
- **DEACON et al.** *16th International Diabetes Federation Congress Abstracts, Diabetologia Supplement,* 1997
- **IRWIN et al.** *Proc. Natl. Acad. Sci. USA,* 1997, vol. 94, 7915
- **MOJSOV.** *Int. J. Peptide Protein Res.,* 1992, vol. 40, 333
- **GÖKE ; BYRNE.** *Diabetic Medicine,* 1996, vol. 13, 854
- **HOLZ ; HAKNER.** *Comp. Biochem. Physiol.,* 1998, vol. 121, 177
- **RITZEL et al.** *J. Endocrinol,* 1998, vol. 159, 93
- **ENG, J. et al.** *J. Biol. Chem.,* 1990, vol. 265, 20259-62
- **ENG., J. et al.** *J. Biol. Chem.,* 1992, vol. 267, 7402-05
- **ENG et al.** *J. Biol. Chem.,* 1990, vol. 265, 20259-62
- **ENG et al.** *J. Biol. Chem.,* 1992, vol. 267, 7402-05
- **RAUFMAN et al.** *J. Biol. Chem.,* 1992, vol. 267, 21432-37
- **SAMBROOK et al.** Molecular Cloning: A Laboratory Manual. Cold Spring Harbor, 1989
- **BARTLETT ; LANDEN.** *Biorg. Chem.,* 1986, vol. 14, 356-377
- **WATERMAN.** *Bull. Math. Biol.,* 1984, vol. 46, 473
- Remington's Pharmaceutical Sciences. Wiley Publishing, 1990